# EUROPEAN PATENT APPLICATION

(11) **EP 4 462 441 A1**
(43) Date of publication of application: **13.11.2024**
(21) Application number: 23315184.4
(22) Date of filing: 10.05.2023
(51) Int. Cl.: G16H 10/60, G16H 15/00, G16H 20/40, G16H 30/20, G16H 40/20

(54) **SYSTEMS AND METHODS FOR INTELLIGENT RADIATION THERAPY**

(71) Applicant: GE Precision Healthcare LLC, Wauwatosa, WI 53226 (US)
(72) Inventor: REUZE, Sylvain, 78530 Buc (FR); GUPTA, Tripti, Waukesha, WI 53188 (US); YI, Hongxiang, Waukesha, WI 53188 (US); LIN, Timothy, Waukesha, WI 53188 (US); MIAN, Fei, Waukesha, WI 53188 (US); KANDALA, Sampath Telikicherla, Marlborough, MA 01752 (US)
(74) Representative: Fennell, Gareth Charles

(57) **Abstract**

Radiation therapy systems and methods are proposed that include an interoperability engine (112), radiotherapy services, and artificial intelligence algorithms. The systems and methods allow for patient (24) medical support. Patients may be diagnosed with medical imaging devices and through intelligent patient intake (412, 500). The interoperability engine (112) can communicate with a variety of data storage, information technology systems and applications provided by first or third parties. The systems and methods may then be used to provide treatment planning and treatment delivery across the care continuum.

## Description

### TECHNICAL FIELD

The subject matter disclosed herein relates generally to systems and methods that utilize hardware and software computer technology for healthcare, including oncology solutions and radiation therapy.

### BACKGROUND

Cancer and cancer treatment is an important area of the healthcare market. Creating systems and methods for improving physician care of cancer patients is important. One area of patient support is through patient intake, diagnosis, and radiation therapy (RT) treatment plans.

Radiation therapy treatment plan development generally employs medical imaging, such as X-ray, computed tomography (CT), magnetic resonance imaging (MRI), and the like. Development of a radiation therapy treatment plan may include multiple steps, including multidisciplinary team discussions (MDT), diagnostic imaging, radiation oncology, patient registration, CT simulation, patient scheduling, data management and/or storage, image registration, segmentation, contour review, planning, plan review and approval, plan quality checks, starting treatment, and follow up care.

Current radiation therapy methods present a range of challenges including complex care coordination, overly manual processes, and disconnected technology systems. Highly complex care decisions, planning, and treatments delivered by a variety of providers may make care coordination complex. Enhanced collaboration may reduce complexity of care coordination by enabling users to work on materials together in real-time, thus reducing time spent waiting for other users to finish tasks. Radiation therapy methods may include time-consuming contouring, treatment planning, and plan approvals, all of which may include frequent interruptions which are a product of manually performing the methods.

Technological systems and methods for improved cross-vendor capability, interoperability, artificial intelligence support, and efficient data exchange are proposed herein.

### BRIEF DESCRIPTION

In accordance with an embodiment, a healthcare system, method, and engine is provided that may include one or more processors; memory; and one or more programs, wherein the one or more programs are stored in the memory and configured to be executed by the one or more processors, the one or more programs including instructions for: patient intake utilizing a user interface, wherein patient intake additionally receives information from disparate information sources comprising at least an electronic medical record (EMR) and a picture archival and communications system (PACS) and can pre-populate parts of the patient intake form based on artificial intelligence analysis of the information from the disparate information sources; workflow management through a medical diagnosis and treatment process based on the patient intake, including providing a user interface displaying the workflow orchestration across the diagnosis and treatment process; trend monitoring for each step in the workflow management, wherein the trend monitoring may provide suggestions in the workflow based on automatic quality reviews related to the trend; and treatment planning based on user input during the workflow of the medical diagnosis and treatment process.

Further, the artificial intelligence analysis of the information from the disparate information sources can include checking the information based on the possible bounds for the related field of the patient intake form. Additionally, the artificial intelligence analysis of the information from the disparate information sources may include searching and matching the patient info with related clinical trials and verifying the patient info fits within the clinical trial guidelines.

The healthcare systems, methods, and engines may further include the automatic quality reviews related to the trend are based on a medical industry quality standard. Further included is an interoperability engine providing operability between various software applications and data storage systems based on industry standards and application specific programming interfaces. Treatment planning may be further provided based on predictive analysis on the data in the system based on the patient intake and workflow management, stored in a non-relational database and utilizing a Bayesian statistics method of predictive analysis. The healthcare systems, methods, and engines may further include pattern recognition between patients based on artificial intelligence analysis of the workflow management of a plurality of patients, and providing user interface suggestions on a display based on recognized patterns.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description.

### BRIEF DESCRIPTION OF THE DRAWINGS

Numerous aspects, implementations, objects and advantages of the present invention will be apparent upon consideration of the following detailed description, taken in conjunction with the accompanying drawings, in which like reference characters refer to like parts throughout, and in which:
FIG. 1 shows a healthcare environment with systems and methods intelligent radiation therapy, according to an embodiment.
FIG. 2A shows a hardware device for a healthcare environment, according to an embodiment.
FIG. 2B shows a block diagram for a computed tomography hardware and software system, according to an embodiment.
FIG. 3 shows a block diagram for a radiation therapy hardware and software system, according to an embodiment.
FIG. 4 shows a healthcare process and block diagram, according to an embodiment.
FIG. 5 shows a tree form process for assisting healthcare decision making, according to an embodiment.
FIG. 6 shows a radiation therapy process, according to an embodiment.
FIG. 7 shows a process for clinical trial matching, according to an embodiment.
FIG. 8 shows a process for quality checking in a healthcare environment, according to an embodiment.
FIG. 9 shows a process for relationship and pattern recognition in a healthcare environment, according to an embodiment.
FIG. 10 shows a process for auto-updating of healthcare software, according to an embodiment.
FIG. 11 shows a schematic block diagram of a sample-computing environment, according to an embodiment.
FIG. 12 shows a schematic block diagram of another sample-computing environment, according to an embodiment.
FIG. 13 shows a schematic block diagram of another sample-computing environment, according to an embodiment.
FIG. 14 shows a schematic block diagram of another sample-computing environment, according to an embodiment.
FIG. 15 shows a schematic block diagram of another sample-computing environment, according to an embodiment.
FIG. 16 shows a schematic block diagram illustrating a suitable operating environment, according to an embodiment.

### DETAILED DESCRIPTION

In the following detailed description, reference is made to the accompanying drawings that form a part hereof, and in which is shown by way of illustration specific examples that may be practiced. These examples are described in sufficient detail to enable one skilled in the art to practice the subject matter, and it is to be understood that other examples may be utilized, and that logical, mechanical, electrical and other changes may be made without departing from the scope of the subject matter of this disclosure. The following detailed description is, therefore, provided to describe an exemplary implementation and not to be taken as limiting on the scope of the subject matter described in this disclosure. Certain features from different aspects of the following description may be combined to form yet new aspects of the subject matter discussed below.

When introducing elements of various embodiments of the present disclosure, the articles "a," "an," "the," and "said" are intended to mean that there are one or more of the elements. The terms "comprising," "including," and "having" are intended to be inclusive and mean that there may be additional elements other than the listed elements.

Systems and methods herein provide central management for various computer-generated medical data and further provides on-the-spot analysis of data in order to determine whether a specific treatment should continue or be adapted. Further provided is a medical computing system which can automatically record each step in the data analysis process during the treatment.

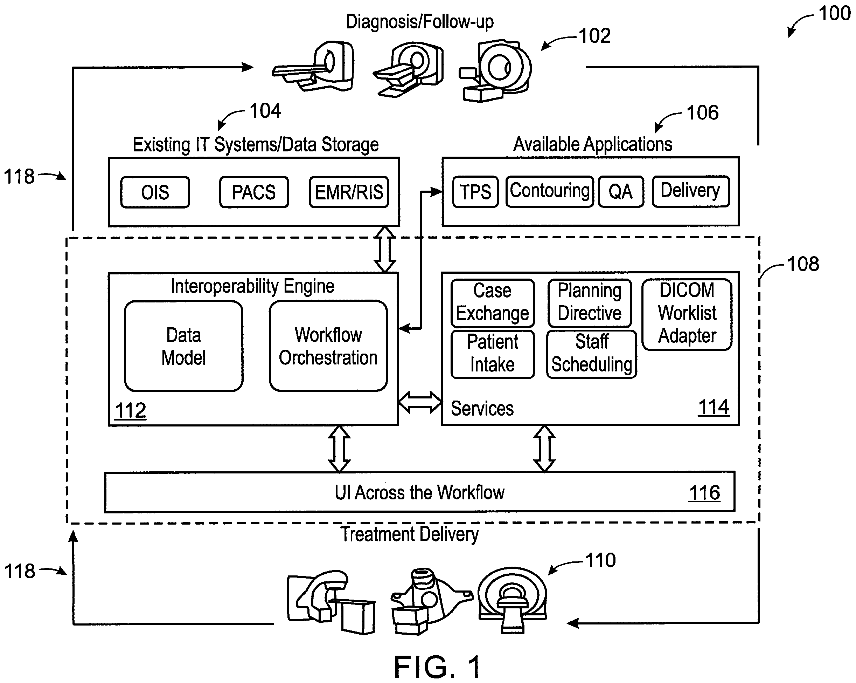

FIG. 1 shows a healthcare environment with systems and methods intelligent radiation therapy, according to an embodiment. FIG. 1 includes oncology healthcare system 100, medical imaging systems 102, information technology (IT) healthcare systems 104, oncology applications 106, radiation therapy (RT) system 108, treatment delivery systems 110, interoperability engine 112, RT services 114, user interface (UI) 116, and data flow 118.

Oncology healthcare system 100 helps patients diagnose and treat medical problems. For example, the oncology healthcare system 100 may be an example of a radiotherapy department. Diagnosis of medical problems may be done with medical imaging systems 102, such as ultrasound, PET/CT, CT, PET/MR, MR, X-ray, and other medical technologies that detect medical problems. Medical imaging data is taken from medical imaging systems 102 and put into existing IT systems/data storage, including IT healthcare systems 102. From these systems, the information can be accessed by compute hardware and software such as RT system 108 and available oncology applications 106 to help decision making, visualization of medical problems, treatment decisions, and more. Interactions across the workflow may be done through UI 116, and treatment may be instructed from RT system 108 to treatment delivery systems 110, such as radiation therapy systems. Then, the patient may return for follow up and further treatment, as indicated by data flow 118.

IT healthcare systems 104 may include at least one of electronic medical records (EMR), picture archiving and communication system (PACS), oncology information system (OIS), radiology information system (RIS), and treatment planning system (TPS). Thus, the TPS application may be an oncology application as shown in FIG. 1, but may also be an existing IT system/data storage of 104. The IT healthcare systems 104 may communicate with the RT system 108. The RT system 108 may include core features and/or services and connectors to host system-accessed applications.

RT system 108 may interact with first party and/or third party oncology applications 106. These applications may be ones on the market that help with tumor contouring, quality assurance, oncology diagnosis, radiation therapy delivery, and more. Interoperability engine 112 connects with available oncology applications 106 to provide interoperability between various IT systems, applications, and the system. Some oncology applications 106 may use one industry standard to communicate, while other applications use another method. RT system 108 through operability engine 112 can interface with a large swath of the technology standards and application programming interfaces in the industry. RT system 108 may access oncology applications 106 via a wired and/or wireless connection. Applications 106 may be deep learning and/or AI applications hosted internally and/or by third-parties, such as OAR contouring- MR, brain tumor contouring MR, synthetic CT (MR only), and/or case exchange and MDT.

RT system 108 may include numerous hardware and software technological functionalities, as discussed herein throughout not just in association with FIG. 1. These interact with and can control the scanning of medical imaging systems 102 and the treatment delivery systems 110, as examples. Technologies with RT system 108 may include interoperability engine 112, RT services 114, and UI 116. As described herein, functions of the RT system 108 include managing patient activities and data in patient radiotherapy treatment course records, as well as operating as a complete workflow management and integration layer for a radiotherapy department in which the RT system 108 is integrated. In some examples, the RT system 108 may use a web-based single-page application to provide users access to patient information from a plurality of sources (e.g., diagnostic imaging systems, patient intake forms, and so on) in a single location. Workflow tasks may be managed by the RT system 108, including stopping and starting workflow applications, display of an execution status of the application, a display of a task owner assigned to an application, and a remainder of a task timeout. For example, the RT system 108 may include containerized micro services which may run on any hardware system, including datacenter, common edge appliance, and cloud implementation.

Management of the workflow tasks may be event-based, such that when data is received by interoperability engine 112, interoperability engine 112 may outpoint a notification, such as to user interface 116, to trigger a customized workflow generated by the interoperability system, which may operate based on different data of the received data. Thus, startup of application of workflows is an event-based mechanism which may be triggered based on data availability and/or receiving user input. The RT system 108 may provide data storage management, including a record of a location, status, and date of operation. Data stored by the RT system 108 may be used to trigger startup of application of workflows.

Using workflow building blocks and information input into the RT system 108, the system may define patient-related workflows and enable the workflows to be adjusted at any time during treatment, subject to versioned workflow definitions/templates. Adjustment of workflows may allow for adaptive radiotherapy, for example, adjusted by a user and/or the interoperability engine or related artificial intelligence based on updated patient, treatment, and/or diagnostic information. As part of deploying workflows, the RT system 108 may trigger launch of at least one external application using DICOM (e.g., MPPS/UPS) or any other protocol for automatic operation of the workflow (e.g., without user input). MPPS stands for Modality Performed Procedure Step. The DICOM Modality Performed Procedure Step (MPPS) closes the loop between the information system, PACS and modality. The modality provides information about the actual performed study, the number of images that were scanned and the status of the exam. As noted further herein, the RT system may include one or more artificial intelligence (AI) module. These may be hardware, firmware, or software. The AI modules may include special purpose Al, machine learning, generative AI, large language models (LLM), neural networks, or other forms of artificial intelligence that takes in data and provides relevant and helpful outputs.

Interoperability engine 112 of the RT system 108, which may include a data model and workflow orchestration module, may interact with existing IT systems and/or data storage, such as OIS (oncology information services), PACS (picture archival and communication systems), and/or EMS/RIS (emergency medical services, radiology information services), as well as existing services including TPS (treatment planning system), contouring, QA (quality assurance), and/or delivery, to exchange patient information. Interoperability engine 112 is an important engine in RT system 108, providing seamless interoperability with existing IT healthcare systems 104, such as OIS, TPS, EMR, radiology information system (RIS), and PACS. The interoperability engine is built on standards, such as DICOM-RT and Integrated Healthcare Enterprise-Radiation Oncology (IHE-RO), to enable collaboration with internal and third-party applications and resources. The interoperability engine provides a structured data flow, automated task triggering, and connectors to multi-vendor RT IT systems. Interoperability engine 112 further enables RT system 108 to connect to third party and first party oncology applications 106.

Interoperability engine 112 uses mechanisms and standards, such as DICOM (Digital Imaging and Communications in Medicine) , DICOM RT, HL7 (Health Level 7), and FHIR (Fast Healthcare Interoperability Resources), which support multiple data protocols, such as a simulation scan, RIS/PACS, EMR (Electronic Medical Record), and OIS (oncology information systems), and further supports routing between different data sources based on a set of rules used to implement functionality of an information exchange hub. The mechanisms and standards may be used to connect the multiple data protocols, such as any EMR and PACS system, with interoperability engine 112 in a vendor-agnostic way, for example, using Integrated Healthcare Enterprise-Radiation Oncology (IHE-RO), DICOM RT second generation, and other standard data protocols. For example, PACS may host an application entity (AE) title and a port number, and an API (Application Programming Interface) may be developed to connect to various PACS following the DICOM standard. Interoperability engine 112 may utilize a back end abstraction layer to connect with external systems in a standard way. The set of rules used to implement functionality of the information exchange hub are clinical tasks used to achieve routing between systems (e.g., between the interoperability system and systems hosting the multiple data protocols). The interoperability engine 112 provides modular workflow tasks to create different rules based on a user request. Information may be exchanged through DICOM and other protocols, such as SOAP (simple object access protocol) and/or REST (Representational State Transfer) , for example, which enables holistic review with integrated data from OIS, TPS (treatment planning system), and RIS/PACS. Information may be exchanged following standard protocols such as DICOM-MPPS, DICOM-UPS, ODBC (Open Database Connectivity, e.g., to connect OIS), and second generation DICOM-RT to connect TPS.

Workflow orchestration module intelligently optimizes workflow designed to eliminate the frustrations of repeated multi-screen data searches. It orchestrates easy access to all applications. Its dynamic data aggregation may be invisible to the user. All clinically related data is automatically collected, analyzed, sorted, and displayed, replacing manual steps that can cost users critical time and effort.

RT system 108 services may include first-party and/or third-party services, including RT services 114 for case exchange, planning directing, tumor-contouring, staff scheduling, patient intake, organ contouring-MR, and/or MR-only workflow (e.g., synthetic CT). Services 114 of the RT system 108 may be hosted and/or provided by internal and/or third parties. These services enable clinical and operational outcomes of the RT system 108. The services may provide for case exchange, patient intake, planning directive, staff scheduling, organ contouring MR, tumor contouring, and MR-only workflow.

Patient intake module and planning directive module is shown in RT services 114. These allow physicians to capture patient information to initiate the patient care journey. Patient demographic information, history, and alerts from the oncology information system can be easily retrieved from one screen through UI 116. The physician's intent of the patient treatment planning are clearly displayed for the team to reference. Planning directive aims to describe the physician's intent of the patient treatment planning goals. Planning directive may also be referred to as plan intent. The patient intake module records and provides a care team of the respective patient with critical information about the patient and their care plan, which may enable the came team to efficiently intake and process the patient. Conventionally, patient intake and processing information is recorded manually (e.g., on paper) during a MDT (multiple disciplinary tumor board) and the information is manually entered into an oncology information system (OIS). A DICOM worklist adaptor is used to provide the following functions to enable patient demographic data and a imaging scanner to retrieve details of patients who are scheduled for scanning from the HIS/EMR/OIS system in the radiation oncology environment. Further, patient intake module allows physicians to capture patient information to initiate the patient care pathway. It can be designed to retrieve patient demographic information, history, and alerts from the oncology information system.

Planning directive module manages the on-call personnel schedule in the department. Its algorithms and processes helps calculate the role-based on-call schedule in a mid- to large-size medical department.

Staff scheduling module in RT services 114 may be used to manage and schedule a workforce of the medical (such as radiation oncology) department based on factors such as staff availability. Planning directive module further enables a radiation oncologist to add detailed prescription digitally for the planning team, which may include a medical dosimetrist and/or medical physicists. The RT system 108 may be communicably coupled to elements of the oncology healthcare system 100 via one or more connectors.

The RT system 108 further includes a user interface (UI) 116, which may be a single UI across the workflow. User interface 116 of the RT system can provide a single UI across the RT care pathway, minimizing touchpoints with existing RT IT systems. The UI provides a workflow view with enabled configurability and action management by users. The RT system 108 may receive, process, and output information pertaining to a patient, a workflow, and at least one resource, as described herein, to automatically guide and execute steps of diagnosis, simulation, treatment delivery, and follow-up.

Treatment delivery systems 110 provide medical treatment to patients. Medical and healthcare decision makers (such as clinicians, physicians, physician assistance, nurses, etcetera.) use RT system 108 to make decisions and assign treatment plans. Healthcare professionals work with treatment delivery hardware and software systems 110 to provide the medical treatment specific to each patient. In an embodiment, this may include medical linear accelerators for external beam radiation treatments for patients with cancer. These can deliver high-energy x-rays or electrons to a malignant tumor in a patient. Further machines may include a combination of a CT scanner and an external-beam radiation machine. Image guided radiation therapy also helps track cancer and spare normal tissues. Other forms of oncology radiation therapy may be intensity-modulated radiation therapy and volumetric modulated radiation therapy. Medical devices in these spaces may be examples of treatment delivery systems 110.

Data flow 118 shows how the data may flow, according to an embodiment. In some examples, the diagnosis-to-treatment workflow operates as a cycle. After treatment, the results of the medical treatment may be fed back to RT system 108, medical imaging devices 102, healthcare IT systems 104, and available applications 106. Further, this results data is fed into artificial intelligence modules within the system for continuous learning.

A modular approach may be used by the RT system 108 to facilitate an exchange of functional modules to support various needs of a radiotherapy department in which the system is integrated. In some examples, the RT system 108 may support needs of a greater environment, for example, a hospital or other healthcare facility in which the radiotherapy department is situated, to enhance collaboration of the radiotherapy department with other departments. When patient data becomes available to the RT system 108 (e.g., via a patient intake module), a CT/MR simulation scan may be scheduled by importing patient demographics and a simulation scan protocol may be generated by importing scan order textual data provided by the patient intake module. A modular workflow may further may defined based on user input.

The RT system 108 may create versioned workflow definitions (e.g., templates) using a radiation oncology domain specific language (DSL) that provides flexible workflow building blocks. The workflow building blocks may contain business logic and interoperability definitions. For example, diagnostic imaging may be performed on a patient and a multidisciplinary team discussion (MDT) may be had regarding the patient. Information from the diagnostic imaging and MDT, as well as patient demographic data and medical alert history may be transferred from a patient intake module and used to schedule a patient scan. The transferred information may be further used to perform image contouring following the simulation scan. Contoured images are transferred to treatment planning system, which may enable users to view contoured images. Interoperability engine 112 working with RT services 114 creates a treatment plan using a plan directive. The treatment plan is transferred to treatment delivery systems 110 following quality review and approval.

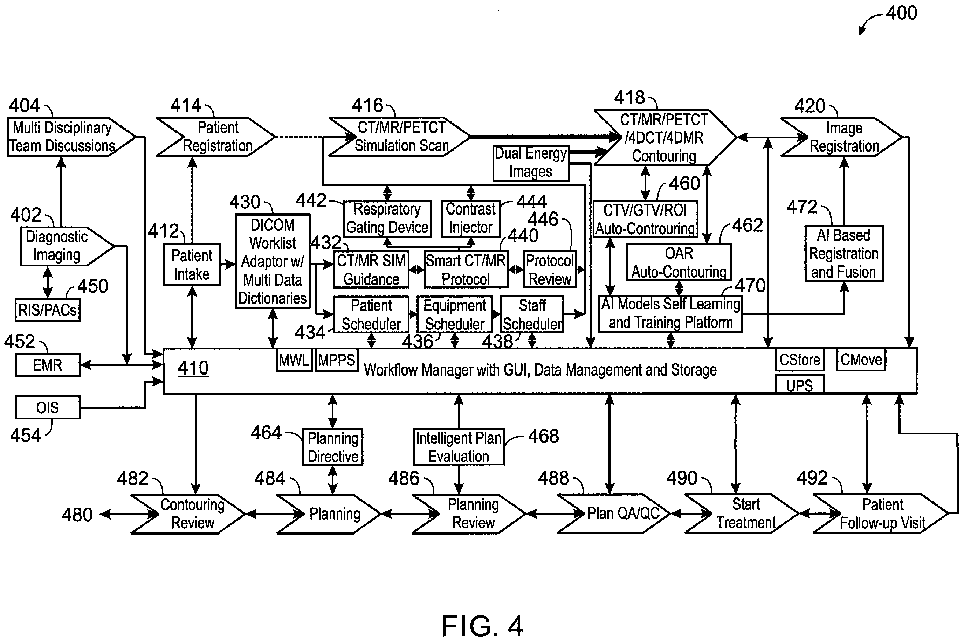

The RT system 108 further includes a workflow orchestration module which enables a care team to orchestrate a treatment planning workflow. This patient-centric, protocolized application manages and monitors a patient treatment course. Thus, the RT system 108 is enabled to automate workflow and minimize manual operation of applications, which may improve efficiency and enhance safety. Further detail regarding the workflow manager is described with respect to FIG. 4.

As shown in FIG. 1, RT system 108 utilizes connector technology to various components such as EMR, imaging devices, imaging and treatment planning software, scheduling and appointment management tools, and quality assurance and monitoring systems for exchanging healthcare information electronically, including messaging, documents, and clinical data. RT system 108 may also incorporate remote monitoring and telemedicine technologies to enable remote consultations and follow-up care.

RT system 108 with API access to supported 3^{rd} party applications can prepare and set the necessary parameters for an application before launching it. This includes available oncology applications 106. By sending this information early, it can save time and effort, as the user does not need to manually configure the application each time it is launched. Instead, the workflow orchestration module of RT system 108 can provide a set of predefined parameters that the application can load and use automatically. This can help streamline healthcare workflows and make them more efficient. Further, RT system 108 can use APIs to launch and access the functionality of each application programmatically, enabling seamless integration with other applications in the workflow. RT system 108 also supports the use of custom scripting to automate repetitive tasks, such as file transfers or data conversions, which further streamlines the workflow process.

The RT system 108 may seamlessly integrate applications with defined and supported standard interfaces in order to execute at least one given task in a clinical workflow. This may also provide easy replacement of given applications with others without impacting an overall workflow. For example, a user may prefer to use a first application instead of a second application, the second application integrated in the overall workflow. The first application may replace the second application in the overall workflow without interruption thereto.

Further, RT system 108 can provide automation for workflow execution with a possibility to configure triggering conditions for orchestration of following steps in each case. RT system 108 can provide an option for users to insert review points which may intentionally stop execution of the workflow, for example, to wait for a user interaction with the workflow. At any point during execution of a workflow, the RT system 108 provides users with an option to review intermediate results of the process, initial data, and accept user input to continue, halt, or roll back execution of the workflow. Users may create a flexible workflow with different instances and/or vendors for a given workflow step. For example, a user may choose a desired application for a given step of the workflow from a catalog of applications. The RT system 108, through interoperability engine 112, provides a framework and interfaces to output, for storage and/or display, configurations, orchestrations, and/or protocols from one user to another, which may provide opportunities for collaboration among users (e.g., of different teams within or outside of a medical department). RT system 108 may provide tools for data and execution analytics, as well as resources for device optimization for workflows and resources for re-adapting the workflow for compatible devices (e.g., when an updated and/or new device is introduced to the workflow).

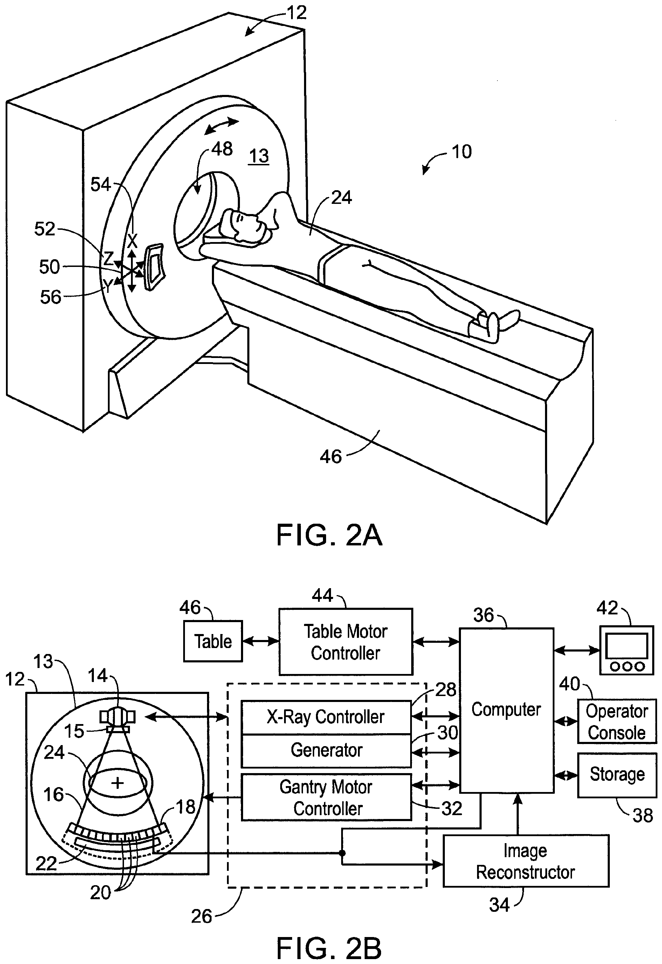

FIG. 2A shows a hardware device for a healthcare environment, according to an embodiment. FIG. 2B shows a block diagram for a computed tomography hardware and software system, according to an embodiment. Such machines may be used as imaging devices 102.

FIGs. 2A and 2B show a computed tomography (CT) imaging system 10 including a gantry 12. Gantry 12 has a rotary member 13. An x-ray source 14 that projects a beam of x-rays 16 through pre-patient collimator 15 toward a detector assembly 18 on the opposite side of the rotary member 13. X-ray source 14 may also be referred to as x-ray tube or x-ray generation component. X-ray source 14 is a type of emissions component. A main bearing may be utilized to attach the rotary member 13 to the stationary structure of the gantry 12. Detector assembly 18 is formed by a plurality of detectors 20 and data acquisition systems (DAS) 22, and can include a post-patient collimator. The plurality of detectors 20 sense the projected x-rays that pass through a subject 24, and DAS 22 converts the data to digital signals for subsequent processing. Each detector 20 produces an analog or digital electrical signal that represents the intensity of an impinging x-ray beam and hence the attenuated beam as it passes through subject 24. During a scan to acquire x-ray projection data, rotary member 13 and the components mounted thereon can rotate about a center of rotation.

Rotation of rotary member 13 and the operation of x-ray source 14 are governed by a control mechanism 26 of CT system 10. Control mechanism 26 can include an x-ray controller 28 and generator 30 that provides power and timing signals to x-ray source 14 and a gantry motor controller 32 that controls the rotational speed and position of rotary member 13. An image reconstructor 34 receives sampled and digitized x-ray data from DAS 22 and performs high speed image reconstruction. The reconstructed image is output to a computer 36 which stores the image in a computer storage device 38.

Computer 36 also receives commands and scanning parameters from an operator via operator console 40 that has some form of operator interface, such as a keyboard, mouse, touch sensitive controller, voice activated controller, or any other suitable input apparatus. Display 42 allows the operator to observe the reconstructed image and other data from computer 36. The operator supplied commands and parameters are used by computer 36 to provide control signals and information to DAS 22, x-ray controller 28, and gantry motor controller 32. In addition, computer 36 operates a table motor controller 44 which controls a motorized table 46 to position subject 24 and gantry 12. Particularly, table 46 moves a subject 24 through a gantry opening 48, or bore, in whole or in part. A coordinate system 50 defines a patient or Z-axis 52 along which subject 24 is moved in and out of opening 48, a gantry circumferential or X-axis 54 along which detector assembly 18 passes, and a Y-axis 56 that passes along a direction from a focal spot of x-ray tube 14 to detector assembly 18.

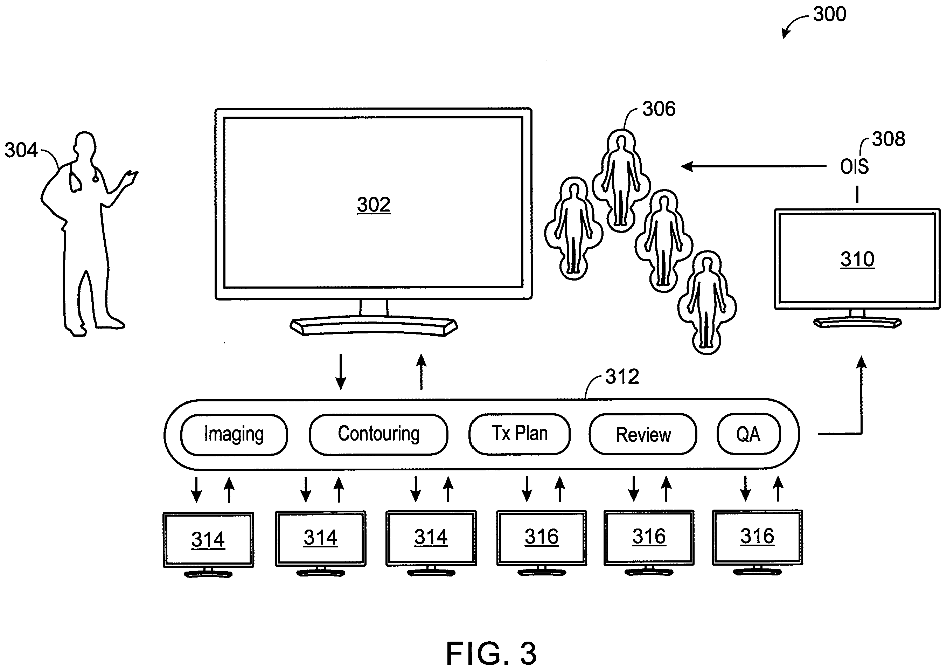

FIG. 3 shows a block diagram for a radiation therapy hardware and software system, according to an embodiment. FIG. 3 includes medical environment 300, display device 302, medical professional 304, RT system 312, first-party medical applications 314, third-party medical applications 316, treatment delivery software 310, oncology information system 308, and patients 306.

RT system 312 provides features and functionality of the RT system 108 as described in relation to FIG. 1. A user interface may be displayed on display device 302 to allow medical professionals 304 to interact with the software and hardware of RT system 312. RT system 312 includes interoperability and software to provide imaging support, tumor contouring, treatment (Tx) planning, review of patient records and treatment plans, as well as quality assurance (QA). Through the interoperability engine, RT system 312 can successfully interact with first party medical applications 314 and third party applications 316. Such interactions may be done by industry standards, application programming interfaces, and/or specific technologies, as discussed herein throughout. First party applications 314 may be those of a company that provide various diagnosis or treatment of medical issues, such as CT, MR, radiation therapy, X-Ray, and other medical software and hardware. Third-party applications 316 are those of third party companies that provide various diagnosis or treatment of medical issues, such as CT, MR, radiation therapy, X-Ray, and other medical software and hardware. The ability of RT system 312 to work successfully with the variety of first and third party applications is valuable for medical professionals 304. Once a medical professional 304 has interacted with RT system 312 to finalize a treatment plan, treatment delivery software 310 works with oncology information system 308 to deliver medical treatment to patient 306.

FIG. 4 shows a healthcare process and block diagram, according to an embodiment. A flow chart 400 is shown which illustrates a workflow of the RT system 108 of the oncology healthcare system 100. Workflow manager 410 of RT system includes graphical user interface (UI), data management, and storage, in an embodiment.

Flow chart 400 shows various steps in a process of medical care, and how a radiation therapy system can provide support for patients. Previous data related to a patient and patient history may come into RT workflow manger 410 from OIS 454, EMR 452, RIS/PACS 450, diagnostic imaging 402 (for example from medical imaging devices), data from image registration 420, and multi-disciplinary team discussions 404. When patient intake 412 occurs, a patient starts, or re-starts, their medical journey and they are registered in patient registration 414. Then, their journey may go through a number of the steps shown in flow chart 400 depending on a number of factors about the patient and medical condition. These steps include contouring review 492 of a tumor, treatment planning 484, planning review 486, plan quality review 488 (QA - quality assurance, QC - quality control), starting treatment 490, and patient follow up visits 492. Modules in the system may help to automatically assist in these steps, such as planning directive module 464 and intelligent plan evaluation 468. Further, depending on the patient and medical issue, additional steps may take place, such as CT/MR/PETCT simulation scans 416, CT/MR/PETCT/4DCT/4DMR contouring 418 - which may use CTV/GTV/ROI auto-contouring 460 and/or OAR auto-contouring 462, image registration 420, CT/MR SIM guidance 432, patient scheduling with patient schedule 434, equipment scheduling through equipment schedule 436, staff scheduling with staff schedule 438, smart CT/MR protocol 440 selection, protocol review 446, contrast injection 444, and AI based registration and fusion 472.

The steps shown in flow chart 400 and workflow manager 410 can utilize AI models self learning and training platform 470. As discussed herein below related to processes and methods of future figures, the RT system leverages machine learning and artificial intelligence for many innovative assistance for the patient journey. Further, such AI models can be continuously trained with further input about the patient and the medical problem.

The workflow manager 410 is an example of the workflow manager and/or the interoperability engine of the RT system 108 of FIG. 1. In the example of FIG. 4, data protocols include a CT/ MR/ PETCT simulation scan 416 (e.g., using MWL and/or MPPS), a CT/ MR/ PETCT/ 4DCT/ 4DMR contouring 418, RIS/ PACs 450, EMR 452, and OIS 454. The workflow manager 410 supports information exchange through DICOM and other protocols, enabling holistic review with integrated data from: OIS 454; treatment planning systems (TPS) including a planning system 484, a planning review system 486, and a plan quality assessment/quality control system 488, EMR 452 and RIS/PACS 450. Textual data is transferred between functional modules (e.g., indicated by blue boxes) and DICOM and DICOM-RT data are transferred between workflow steps (e.g., indicated by brown boxes).

During a multi-disciplinary team discussion 404, patient images and documents are exchanged among team members. Conventionally, patient information may be physically recorded (e.g., on paper) during a multi-disciplinary team meeting (MDT) (e.g., the multi-disciplinary team discussions 404) and be manually input into an oncology information system (OIS) 454. As described herein, the workflow manager 410 securely manages distribution of patient images via an images interface (e.g., DICOM) and distribution of documents via a documents interface (e.g., textual-based communication), such as HL7. For example, results and/or conclusions may be distributed between healthcare providers across geographical distance, network boundaries, and/or through a professional social network.

Patients who are confirmed to undergo radiotherapy treatment register with the radiotherapy department, which may include providing patient information such as medical history, demographic information, collectively information about a patient condition, and so on to clinical staff. The workflow manager 410 guides clinical staff through filling in a patient intake 412 according to a predefined workflow. For example, some patient information may be used for the patient intake 412 for a first workflow and not used for a second workflow. Through a DICOM worklist adaptor 430 provided by the workflow manager 410, information exchange and automatic populating a workflow with radiotherapy imaging equipment from different manufacturers may be realized by the patient intake 412, including patient demographics, a scanning schedule, and specific scanning notes and requirements, for example. The patient intake 412 may provide a care team of the patient with information about the patient and a care plan for the patient to enable the care team to efficiently intake and process the patient.

The DICOM worklist adaptor 430 may be configured with multiple data dictionaries which are publicly released and/or authorized by different manufacturers. For example, the DICOM worklist adaptor 430 is configured as a converter which loads data dictionaries of manufacturers with common rules, adapts the data dictionaries to match data languages, and transfers patient intake information into a language used by the scan device. In this way, the DICOM worklist adaptor 430 may convert non-standardized updated information into a standardized format. Conventionally, DICOM worklist adaptors and demographic data may be used mainly for radiology. In a radiation oncology department, an oncology information system (OIS) manages and stores specific oncology demographic information. The interoperability system also includes modality work list (MWL) services. Patient demographic data transfer between MWL services provided by OIS may pose a challenge, as different vendors may use different DICOM tags in OIS, which may result in data in OIS not being fully recognized by CT or MR systems. The DICOM worklist adaptor 430 of the interoperability system disclosed herein may allow CT and/or MR simulation scanners to parse specific oncology information which is supported by the respective simulation scanner and is transmitted via the DICOM modality worklist adaptor 430.

The workflow manager 410 may seamlessly connect DICOM Tag data via the DICOM worklist adapter 430 by loading the multiple data dictionaries and using data matching algorithm contained therein to provide interoperability of cross-platform and cross-manufacturer data communication. For example, in conventional systems, patient data registered in the OIS system 454 may not be completely transferred to a simulation CT scanner, as further described herein. A user (e.g., care provider) may manually enter patient demographic information and an additional user may cross-check entered information to ensure accuracy of entered patient data (e.g., information about the patient's condition) during patient registration 414. This process may be time-consuming and laborious, thus a method for automatically populating a patient intake form with patient information is desired for enabling fast and accurate transmission of information. Use of the DICOM worklist adaptor 430 together with the patient intake 412 may significantly reduce a current overly manual process and reduce a possibility of manual error.

Using the patient intake 412, a patient simulation scan order may be generated using a CT/MR simulation guidance module 416. A simulation scan may follow standard operation of conventional modality scans, such as CT, MR, and PET-CT scans. In some examples, a simulation scan may be a computer simulated scan which outputs a predicted image which may be generated by the scan based on input parameters. Operation of each module of the simulation scan is the same as conventional module operation, the interoperability system described herein enables coordination among modules. Compared to conventional simulation scanning, the simulation scan directed by the patient simulation scan order may include a series of decentralized factors, including patient registration, patient appointment, scanning protocol creation, equipment preparation, operator preparation, and so on, enabling independent management of the process. Enabling individual modules of the interoperability system to run dynamically through a coordinated mechanism may allow for generation of a new patient appointment list when a patient appointment is changed, as described with respect to the patient scheduler 434.

Data preparation for a simulation scan 416 is managed through workflow modules to automatically export simulation scan modalities. Generation of the patient simulation scan order may include using the patient intake 412 module to collect relevant patient demographic and care information. The interoperability and workflow manager system may then initiate workflows within a radiation oncology department (an example department in which the interoperability system is integrated). Enterprise-wide information systems, such as one or more EMR systems 452, may be used to collect radiation oncology patient demographic data, disease specific information, and simulation scan order information such as scan area, contrast agent, and so on. The interoperability and workflow manager system may output data forms in an electronic format (e.g., a standardized format) compatible with simulation scan systems (e.g., CT, MR, PET/CT, PET/MR) via the DICOM worklist adaptor 430. The patient simulation scan order may indicate that a patient simulation scan has been requested and/or recommended by a healthcare provider and/or based on patient information of the patient intake 412.

Workflow manager 410 may automatically generate recommended scan protocols for a simulation image device (e.g., CT, MR, PET CT, etc.) through an intelligent scan protocol module 440. The intelligent scan protocol module 440 may obtain patient demographic information (e.g., weight, height, age, gender, etc.) from the patient intake 412 and generate a protocol in combination with scan protocol image device guidelines which comply with industry standards and/or user-defined guidelines.

Workflow manager 410 further includes a protocol review and approval mechanism 1205 which is used to review the scan protocol(s) generated by the intelligent scan protocol module 440 to determine if an appropriate scan protocol/sequence has been selected. The intelligent scan protocol module 440 is configured with algorithms which can generate matching parameters for respiratory gating devices 442 and contrast agent injectors 444 from the scan order. For example, machine learning based models are used as algorithms to drive automatic generation of scan protocols. Selected protocol data which is stored in the interoperability system may be obtained from a prior scan. The selected protocol data is used as a basis for future procedure recommendations and scan protocol planning. Each selected protocol includes a RIS procedure name (e.g., as defined in the MWL), a scanner protocol identifier, a patient age, a patient height, and a patient weight, as well as parameters for respiratory gating devices and contrast agent injector. Recommended scan protocols may be performed in at least one of two ways: a linear weighted moving average (LWMA) statistical technique or a machine learning technique, such as a naive bayes classifier. Operating parameters for each of respiratory gating device 442 and contrast agent injector 444 are sent to the respective device by the workflow manager 410.

Following protocol generation, an administrative process and user interface may be used to display the generated protocol. In protocol review 446, users may provide user input which may be used by the interoperability system to change steps of the generated protocol. For example, the generated protocol and prior protocols and/or alternate generated protocols may be output for display such that more than one protocol may be displayed for comparison at once. Display of protocols may include display of embedded electronic scanning guidelines. In protocol review 446, an operational interface may be provided to enable a user to approve or change the generated protocol. Following receipt of a user input indicating approval of the protocol, the interoperability system executes the generated protocol by starting the next step in the workflow.

A patient scheduler module 434 uses Al algorithms to view and predict a probability of a patient no-show and/or delay over a period of time. For example, the period of time may be seven days. Based on the predicted probability, the patient scheduler module 434 may automatically generate an updated patient schedule based on patient priority and availability in the event of a patient no-show or delay. Thus, as part of workflow manager 410, patient scheduler module 434 may automatically provide guidance to the patient for scan and treatment preparation based on the simulation scan order and subsequent radiotherapy treatment plan. For example, workflow manager 410 may generate a calendar notification to be sent to the patient which includes a time, date, location, and requested preparation for the scan. Patient response feedback may be provided to workflow manager 410 via patient scheduler module 434, such as an availability of the patient to undergo the scan.

Workflow manager 410 is further configured with equipment scheduler module 436 which can automatically coordinate an equipment schedule of a department (e.g., a radiotherapy department including at least one scan device). Equipment scheduler module 436 may coordinate scheduling of equipment in the department, including at least one simulation device, TPS, and treatment deliver device such as linac proton therapy (high-energy radiation that may be used to treat cancer). Equipment scheduler module 436 may further automatically update the equipment schedule as patient schedules change, based on equipment status, and a priority to align with a latest patient schedule.

Staff scheduler module 438 of the workflow manager 410 may automatically manage and schedule a workforce of the medical department based on factors such as staff availability, procedures upcoming, geographic details, and more. Thus, workflow manager 410 may automatically identify, plan, and schedule a patient scan using the patient scheduler module 434, the equipment scheduler module 436, and the staff scheduler module 438. Coordination of patient, staff, and equipment may be self-adapted by the workflow manager 410. Relevant personnel may be informed, such as via text message and/or e-mail, of a time, date, and location of the scan. Thus, the workflow manager 410 provides a method which effectively ensures and improves operational efficiency among departments.

Upon completion of the above steps to intake a patient, identify parameters for a scan, and schedule the scan, the workflow manager 410 automatically coordinates a CT/MR/PET_CT simulation scan 416. The simulation scan includes corresponding patient information, scan protocol, patient schedule, equipment schedule, and staff schedule, as described above. The simulation scan 416 is performed and, following completion of the simulation scan, workflow manager 410 obtains information about the end of the simulated scan via DICOM MPPS, in an embodiment.

Patient image data generated by the simulated scan is automatically transferred to a contouring application 418. Workflow manager 410 may manually control a start and a stop of a contouring procedure performed by the contouring application 418, or may automatically trigger the contouring procedure to start by transferring the patient image data to the contouring application 418. For example, a workflow engine of workflow manager 410 uses availability of patient image data to automatically trigger OAR auto-contouring 462 and/or AI models self-learning and training platform 470. A hardware platform and AI model training software of the self-learning and training platform 470 may allow users to train contouring AI models and update existing models for continuously improving results. Exported data from the contouring application 418 is temporarily saved in a database of workflow manager 410 and routed to an image registration process 420 to automatically launch an image fusion operation 472. For example, if the patient undergoes multi-modality simulated scans, such as CT, MR, and PET_CT scans, image fusion between differently contoured outputs is performed. Availability of image data may trigger generation and display of a notification on a user interface. Based on the notification, a user may interact with the user interface to provide user input. Receipt of user input may indicate start or stop of auto-contouring provided by AI algorithm.

Following the image registration process 420, the patient image may undergo contouring review 482, as performed by a user and/or an automated system. Based on the contouring review 482, TPS is performed which includes planning 484, planning review 486, and plan QA/QC 488. Workflow manager 410 includes a digital planning directive application 464 as part of planning 484, which intelligently assists a user (e.g., a radiation oncologist, a medical dosimeter, and/or a medical physicist) in developing a radiation treatment plan and generating a digital, detailed prescription to replace a current paper radiotherapy plan. For example, the planning directive may include information regarding a physician or other healthcare provider's radiation treatment planning goal, which may be used to improve efficiency and communication of the treatment planning process by enabling standardized communication. Input of the planning directive application 464 may be a pre-set template of planning goals for a patient according to a plan selected and manually populated by healthcare providers. Plan information may be output for display to users and further saved to a database of the interoperability system. The plan may be reviewed by planning review 486, which includes an intelligent plan evaluation application 468 that uses AI techniques to pre-evaluate the treatment plan and suggest modifications. At the same time, the evaluation module based on AI techniques may use a self-training platform provided by the workflow manager 410 to allow users to train plan evaluation AI models and update existing models for better results. The treatment plan is checked for quality assurance and quality control at plan QA/QC 488.

Once the treatment plan has been approved by the treatment planning steps, workflow manager 410 may provide the radiotherapy department with information used to start treatment 490. For example, the information includes the generated and approved treatment plan, registered/fused/contoured patient images, patient demographic data, and so on. Following treatment, a patient follow-up visit 492 may be scheduled to discuss treatment results and/or determine future treatment plans. Changes to the treatment plan and treatment result data may be input into the workflow manager 410 to assist in tracking patient progression through the treatment plan.

In this way, the workflow manager of an RT system provides automation, allowing users to configure workflows and automatically orchestrate data integration, task execution, and result deployment while connecting with multi-vendor external systems. Third-party applications may be controlled and launched by calling their interfaces for AI/DL applications in a standard interface. The system may provide an extensive catalog of powerful radiation oncology applications and provides seamless integration of any application within a unique onboarding process. The system may provide a single user interface to be used with existing RT IT multi-vendor systems. The system may store only reference data, reducing data overhead. The system may further reduce a cognitive burden on users caused by navigating multiple software systems.

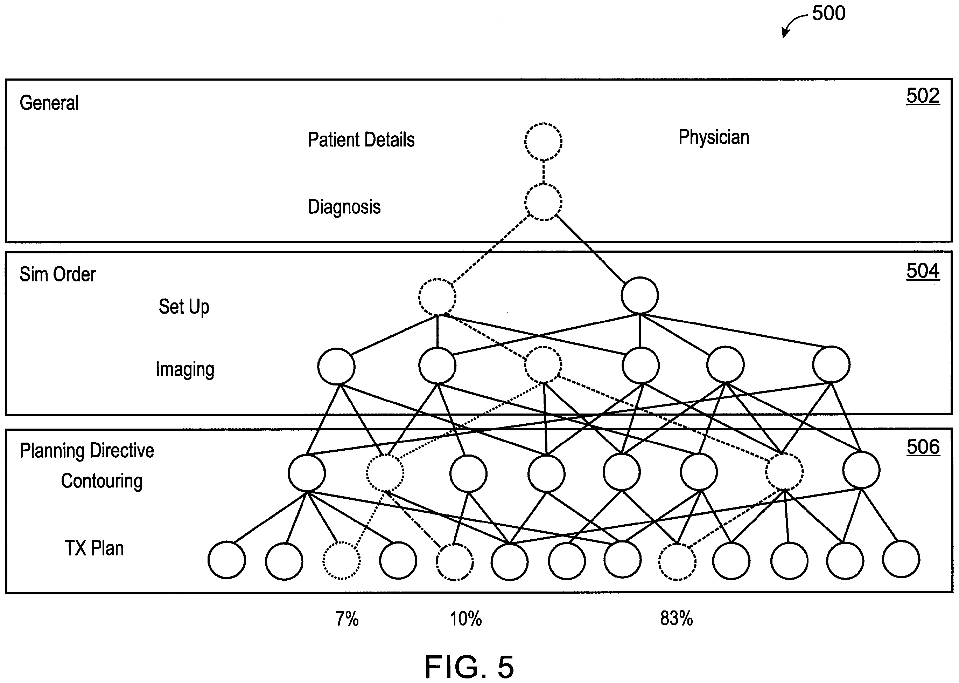

FIG. 5 shows a tree form process for assisting healthcare decision making, according to an embodiment. This may also be referred to as a multi-level model. FIG. 5 includes RT system patient intake 500, general block 502, sim order block 504, planning directive block 506.

In one embodiment, most likely treatment plans for an individual may be determined by a non-relational database and Bayesian statistics method, as shown in patient intake 500 example. Compared to the relational database, the non-relational database detaches the data from application and stores the data in a tree structure. This allows flexible schema in which Bayesian statistics will be used to calculate the right data path. Bayesian statistics utilize observed and unobserved parameters in a statistical model for probability distribution. Such Bayesians network are probabilistic graphical models that represent a set of variables and their conditional dependencies via a directed acyclic graph (DAG), such as the example of FIG. 5. Bayes' theorem describes the conditional probability of an event based on data as well as prior information or beliefs about the event or conditions related to the event, such as medical treatments for patients. The dashed outlined circles and lines in FIG. 5 indicate an initial probability flow through the tree structure based on factors such as patient details, diagnosis, and set up. At the imaging layer, the predictive Bayesian statistics diverge for the dashed outline (most probable line) and the dotted outline paths. And further, at the contouring level, when we learn more about the tumor through contouring, the predicted paths diverge again adding the dashed and dotted outline. As shown in the example of FIG. 5, the predictive capabilities of the RT system predicted an 83% likelihood treatment plan with the dashed outline, a 10% likelihood treatment plan with the dashed and dotted outline, and a 7% treatment plan with the dotted outline. Thus, a non-relational data structure and Bayesian model together can help predict the care path for the patient. Once the physicians fill out forms including the variable path for patients, the data can be used to predict the path for future patient. Such non-relational data structures allow this possibility with other models than Bayesian network models, Bayesian network model is an example in an exemplary embodiment.

Patient intake is an important medical area that has needed technical improvement. AI can help fill out patient intake forms automatically, such as medical history, current medications, and allergies. This saves time and reduces human error. An AI assisted smart patient intake form can be used to initiate the medical workflow to the radiation therapy treatment. This form collects patient medical history, current medications, allergies, and other vital information needed to determine the best course of treatment. This form can be automatically completed by the RT system and reviewed by the radiation oncologist and the medical team. When automatically completed, artificial intelligence analysis of the information from the disparate information sources (EMR, PACS, oncology applications, etcetera) includes checking the information based on the possible bounds for the related field of the patient intake form.

As part of patient intake, the systems and methods herein may store templates, and allow storing and searching for a user's preferred list of templates. When using patient intake, the AI can provide an assisted mode. This mode provides that as the user answer questions, the form suggests answers based on most likely response. The user may verify or change values as needed. Further, in additional modes, as the user answers questions, the form suggests key questions that may result in auto-completion of the form. OIS results may provide enough for completion of for a diagnosis, in some cases. This functionality allows workflow and forms to be auto-completed or auto-structured, providing accuracy and efficiency for users. For example, AI/ML can help identify patients who may be at risk for certain side effects or complications and recommend appropriate treatment templates. Such a system introduces workflow efficiency and reduces human error by introducing plausibility functions including predictive workflow.

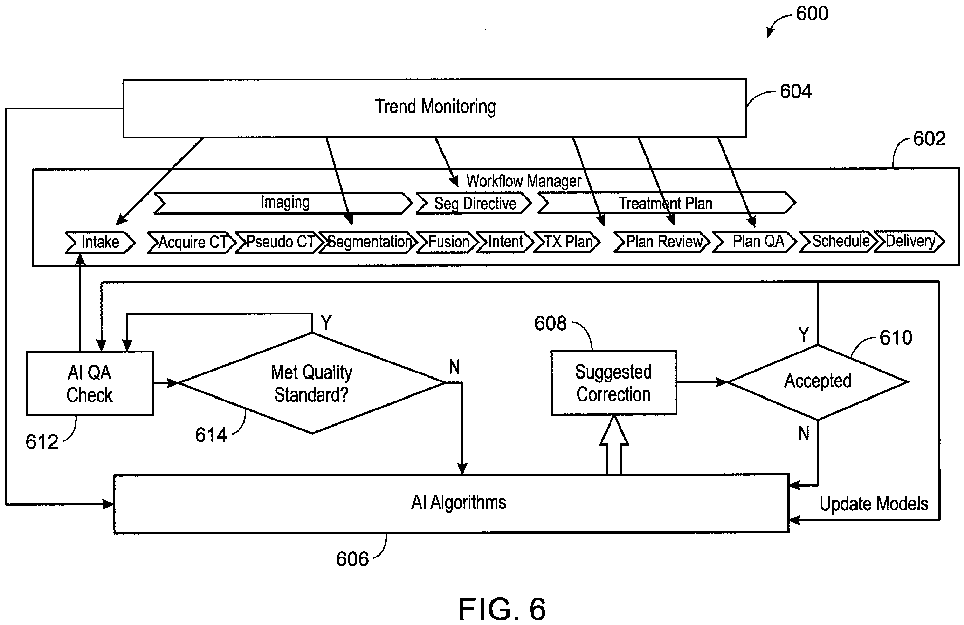

FIG. 6 shows a radiation therapy process, according to an embodiment. FIG. 6 includes AI enabled RT system 600, workflow manager 602, trend monitoring 604, AI algorithms 606, suggested corrections 608, decision step 610, AI QA check 612, and quality decision step 614.

AI enabled RT system 600 promotes workflow data integrity and quality assurance. The workflow manager 602 of RT system 600 includes much of the steps discussed already herein. Allowing for patient intake, acquiring imaging scans, segmenting images, contouring and medical image analysis to understand medical issues in regions of interest, developing treatment plans, scheduling treatment, and delivery of treatment. Throughout this process, RT system 600 includes trend monitoring 604. This monitors trends along each step of the process for AI learning. This includes software trends, such as what parts of the software are most clicked on, which options are most chosen, which parts of the workflow are skipped the most by users, and similar trends. The software can take these trends and update to improve the RT system 600 and workflow manager 602. Additional trends for trend monitoring 604 are medical trends. These include trends related to patient intake, imaging protocol selections, diagnosis, radiology report construction, as well as treatment planning and delivery.

Trend monitoring 604 then feeds these trends and learnings into AI algorithms 606. AI algorithms 606 make suggested improvements and corrections 606 to the software, the medical diagnosis, the treatment plan, and other trends as mentioned herein throughout. The acceptance in step 610 may be done by the system in some cases, for example comparing a change to medical guidelines for that disease and accepting the change if within medical guidelines. In other instances, the acceptance in step 610 may be by a user, for example in changing a dose of radiation therapy in the treatment plan, a physician may review and properly review. Then the changes are quality checked based on software standards, usability standards, medical standards, and the like. If the change meets quality standards, it maybe introduced from AI QA check 612 to workflow manager 602. Throughout the process, AI models and algorithms may be continuously updated based on trends, feedback, decisions by users, as well as data related to quality checks.

The system can utilize Al-powered machine learning algorithms to establish an internal QA system to check the data integrity between work steps and perform quality assurance for each work step across all stags of the workflow. This internal QA system can identify and correct any errors or inconsistencies in the data, ensuring that the data is accurate, reliable, and consistent across all stages of the workflow. Additionally, by utilizing AI and machine learning, the software can learn and improve over time, becoming more efficient and effective at detecting and resolving issues in the workflow. This can ultimately lead to improved patient outcomes and better overall healthcare delivery. AI algorithms 606 can learn from previous quality checks and identify patterns that indicate potential issues, allowing for more accurate and efficient quality control. AI enabled RT system 600 provides real-time monitoring capability by using Al-power algorithms. Trend monitoring 604 monitors the system continuously monitor workflow processes, identifying quality issues as they arise and allowing for immediate remediation.

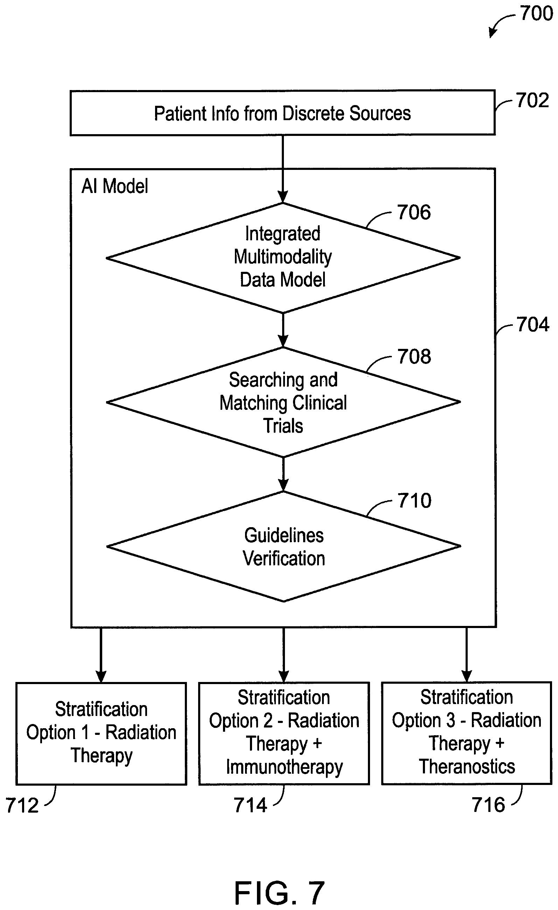

FIG. 7 shows a process 700 for clinical trial matching, according to an embodiment. This process allows for matching patients with appropriate clinical trials or treatment choices based on their medical history and other relevant factors. In step 702, patient information is collected from discrete sources, such as EMR, PACS, and patient intake. These are fed into the AI model 704. In step 706, the patient info is processed through an integrated multi-modality data model. In step 708, the process searches and matches patients up with clinical trials related to their patient information, which may include their diagnosis and treatment plans. In step 710, Al model 704 verifies that the suggestions out of step 708 for matching a patient with a clinical trial are accurate to the relevant guidelines. Then, depending on the AI model process, different stratification output options are selected. These may be option 1 for radiation therapy 712, option 2 for radiation therapy and immunotherapy 714, and/or option 3 for radiation therapy and theranostics. Such a treatment approach with theranostics is a two-pronged approach to diagnosing and treating cancers using radiotracers. Radiotracers are compounds made of radiation and chemicals that selectively bind to a specific target in the body.

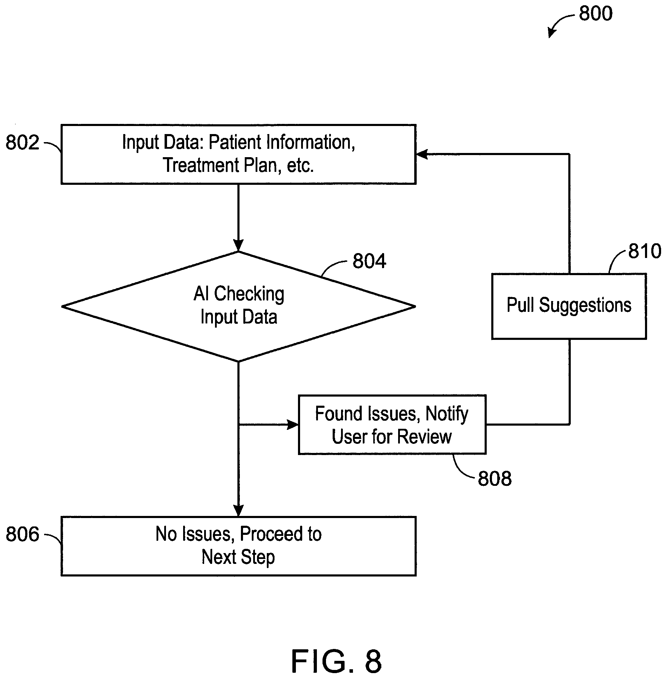

FIG. 8 shows a process for quality checking in a healthcare environment, according to an embodiment. Process 800 allows the RT system to analyze patient data and identify potential issues or outliers in treatment plans or other aspects of care. For instance, identifying a potential error with applying prostate cancer treatment processes to female patients or adult treatment template to pediatric patients. In step 802, input data is retrieved, such as patient information, treatment plan, imaging scan information, and additional information as discussed herein. In step 804, the system checks the input data for quality and accuracy issues through artificial intelligence. For example, if a name is input wrong, or if some input data is outside a possible range for the data, it can identify the issue (e.g. blood pressure is set outside humanly possible ranges, there may have been an input error). If no issues are found, then the process moves to step 806 and the software continues forward in its workflow management process. If issues are round, then a user is notified in step 808. Alternatively, depending on the issue, the system may check with other AI applications to find solutions to the issue automatically. The AI algorithms supporting the RT system may automatically generate specific solution suggestions in step 810 to solve the issue identified. Once selected, the issues are corrected and the process moves back to step 802.

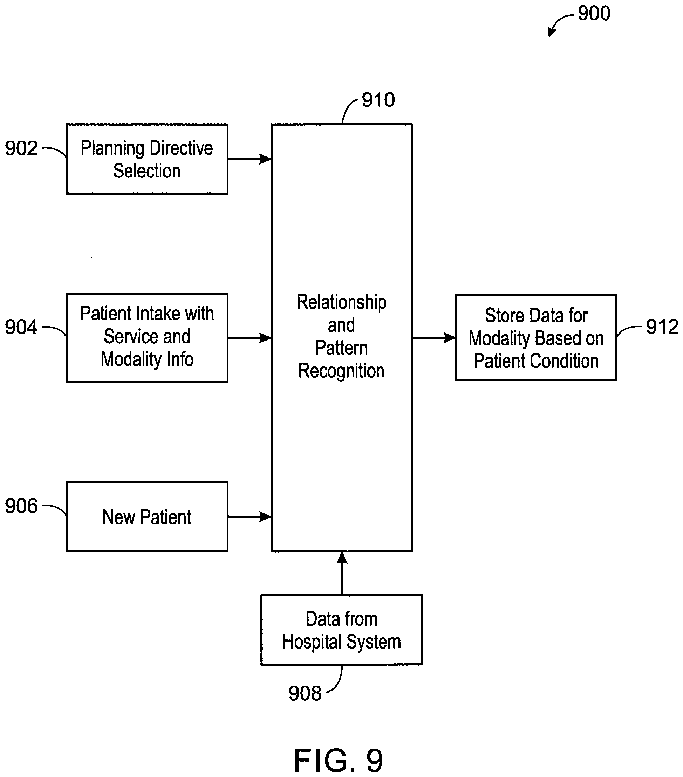

FIG. 9 shows a process for relationship and pattern recognition in a healthcare environment, according to an embodiment. Process 900 allows for predictions based on pattern recognition, which can aid in the decision-making process and to suggest future treatment plan. AI relationship and pattern recognition 910 can help identify patients who may be at risk for certain side effects or complications and recommend appropriate treatment templates. AI relationship and pattern recognition 910 takes inputs from planning directive selection 902, patient intake with service and modality info 904, new patient 906 information, and/or data from the hospital system 908. Relationships or patterns between certain planning directives and medical issues may detected by AI relationship and pattern recognition 910, as an example. Relationships or patterns between certain age and/or gender patients may be detected by AI relationship and pattern recognition 910. These relationships and patterns may be stored data for the modality based on the patient condition in step 912. Alternatively the system make changes to the treatment plan, imaging protocols, software, and or other decisions based on relationship and pattern recognition 910.

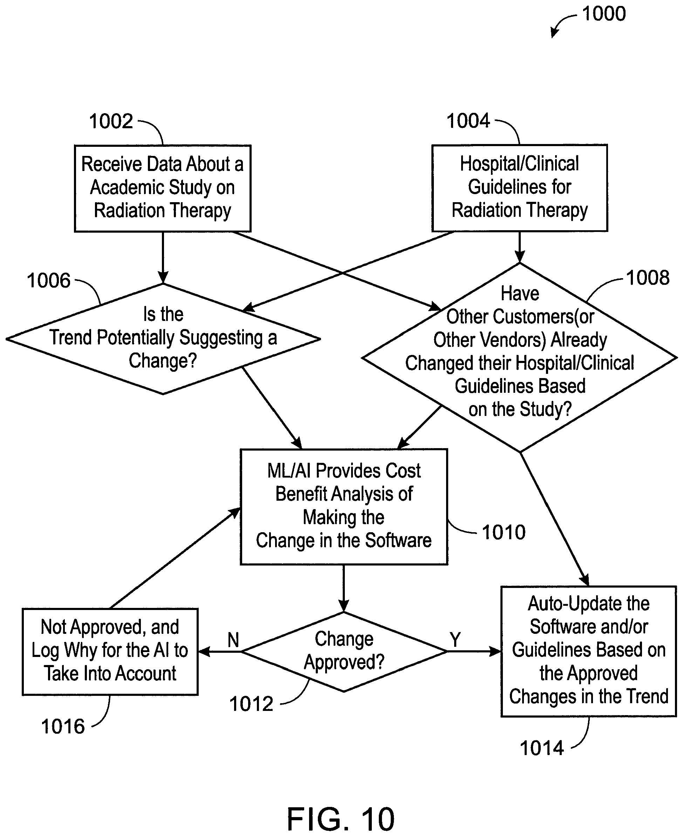

FIG. 10 shows a process for auto-updating of healthcare software, according to an embodiment. The RT can utilize Al-powered machine learning algorithms to process and analyze data from patients and users, uncovering patterns, relationships, and trends. These may be done through AI models self learning and training platform 470, for example. Process 1000 shows how AI can update RT system software and medical guidelines based on current treatments and patients, as well as trends in the industry.

In step 1002, the system receives data about an academic study on radiation therapy. This is one example, but other medical studies, guidelines, and government reports can be included. There is new information related to the medical issue of patients, and the RT system allows for automatic improvement of the medical decisions and related software through this process. The hospital or clinic may have its own related guidelines or rules related to the medical issue or radiation therapy. In step 1004, these guidelines are provided to the process.

In step 1006, the RT system evaluates, based on guidelines from step 1004 and the new trend information from step 1002, whether the trend is potentially suggesting a change to the hospital guidelines or clinician recommendations. In step 1008, the RT system also evaluates whether others have already altered there practice based on the trends, such as changing their hospital or clinical guidelines based on the study. These factors from step 1006 and step 1008 are provided to step 1010.

In step 1010, the AI provides a cost and benefit analysis of making the change in the software and/or treatment plan, as examples. Then the change may be approved (by a user or the system, depending on the type of change) in step 1012. If approved, the software may auto-update itself if the change is related to software, in step 1014. If the change approved is related to the guidelines for the medical issue, or treatment plans, the RT system software can update these as well. If the change is not approved, then the system can log why for the AI to take into account for its own self-learning in step 1016, and this is provided back to step 1010 for learning purposes. Through this process, the system can more quickly improve its efficiency and useability of the software. Additionally through this process, the medical care for patients is more quickly updated and improved based on new knowledge.

The benefits of the methods and systems herein include improved integration, automation, standardization, and personalization, compared to conventional methods for compiling workflow steps. The methods and systems herein enable a resource for patient case review, decision support, and reporting. Managing and scheduling workforce, such as within a radiation oncology department, may be done through the platform based on factors such as staff availability. The methods and systems herein may enable users to achieve better precision, efficiency, integration, ease of use, and patient centric care. Additionally, a time spent with complex workflow steps may be reduced by automation of the workflow steps and execution thereof. The methods and systems herein further provide support for automated workflows and triggering. For example, organ at risk contouring may be automatically triggered and performed. The methods and systems herein may be integrated and interoperable with an existing clinical environment, such as TPS, OIS, HIS, and so on. Multi-vendor clinical interoperability may be enabled and, along with the benefits listed above, may compress a timeline of the RT workflow and provide a better overview, management, and monitoring of the treatment planning workflow. Further, patients and software users benefit from the analysis of staff and equipment utilization (trend monitoring of FIG. 6 as example), individual user behavior and productivity, and treatment process efficiency with assigned users. Further, the systems and methods herein optimize and accelerate data transfer between multiple platforms, reduce the risk of human error, and track the patient along the whole radiotherapy pathway.

The methods and systems herein solve the challenge of data transfer between multivendor infrastructures and allows a fully digital tracking of the patient along multiple appointments. The methods and systems herein add a level of automation in the patient workflow of a radiotherapy department. Through various connectors, the methods and systems herein allow automated data transfer between various 3rd party applications and provides a clear view of patient workflow as well as a universal environment for review and approval at each step of the workflow.

The systems and processes described below can be embodied within hardware, such as a single integrated circuit (IC) chip, multiple ICs, an application specific integrated circuit (ASIC), or the like. Further, the order in which some or all of the process blocks appear in each process should not be deemed limiting. Rather, it should be understood that some of the process blocks can be executed in a variety of orders, not all of which may be explicitly illustrated in this disclosure.

The illustrated aspects of the disclosure may also be practiced in distributed computing environments where certain tasks are performed by remote processing devices that are linked through a communications network. In a distributed computing environment, program modules can be located in both local and remote memory storage devices.

Moreover, it is to be appreciated that various components described in this description can include electrical circuit(s) that can include components and circuitry elements of suitable value in order to implement the embodiments of the subject innovation(s). Furthermore, it can be appreciated that many of the various components can be implemented on one or more integrated circuit (IC) chips. For example, in one embodiment, a set of components can be implemented in a single IC chip. In other embodiments, one or more of respective components are fabricated or implemented on separate IC chips.

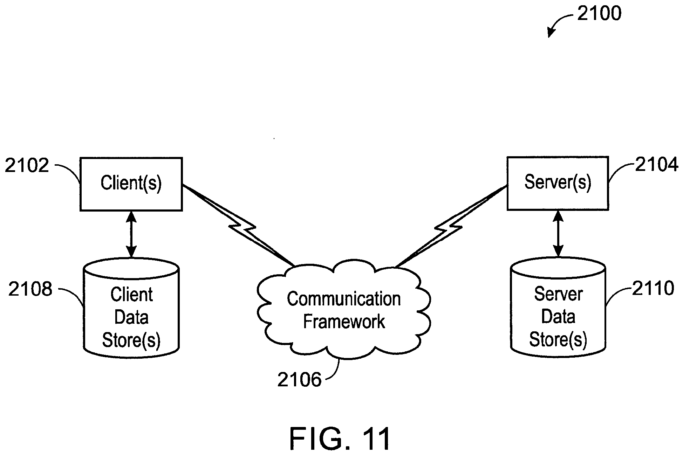

Referring to FIG. 11, there is illustrated a schematic block diagram of a computing environment 2100 in accordance with this disclosure in which the subject systems, methods and computer readable media can be deployed. The computing environment 2100 includes one or more client(s) 2102 (e.g., laptops, smart phones, PDAs, media players, computers, portable electronic devices, tablets, and the like). The client(s) 2102 can be hardware and/or software (e.g., threads, processes, computing devices). The computing environment 2100 also includes one or more server(s) 2104. The server(s) 2104 can also be hardware or hardware in combination with software (e.g., threads, processes, computing devices). The servers 2104 can house threads to perform transformations by employing aspects of this disclosure, for example. In various embodiments, one or more of the subject front end-components can be deployed as hardware and/or software at a client 2102 and one or more of the subject back-end components can be deployed as hardware and/or software at server 2104. One possible communication between a client 2102 and a server 2104 can be in the form of a data packet transmitted between two or more computer processes wherein the data packet may include video data. The data packet can include a metadata, e.g., associated contextual information, for example. The computing environment 2100 includes a communication framework 2106 (e.g., a global communication network such as the Internet, or mobile network(s)) that can be employed to facilitate communications between the client(s) 2102 and the server(s) 2104.

Communications can be facilitated via a wired (including optical fiber) and/or wireless technology. The client(s) 2102 include or are operatively connected to one or more client data store(s) 2108 that can be employed to store information local to the client(s) 2102 (e.g., associated contextual information). Similarly, the server(s) 2104 are operatively include or are operatively connected to one or more server data store(s) 2110 that can be employed to store information local to the servers 2104.

In one embodiment, a client 2102 can transfer an encoded file, in accordance with the disclosed subject matter, to server 2104. Server 2104 can store the file, decode the file, or transmit the file to another client 2102. It is to be appreciated, that a client 2102 can also transfer uncompressed file to a server 2104 and server 2104 can compress the file in accordance with the disclosed subject matter. Likewise, server 2104 can encode video information and transmit the information via communication framework 2106 to one or more clients 2102.

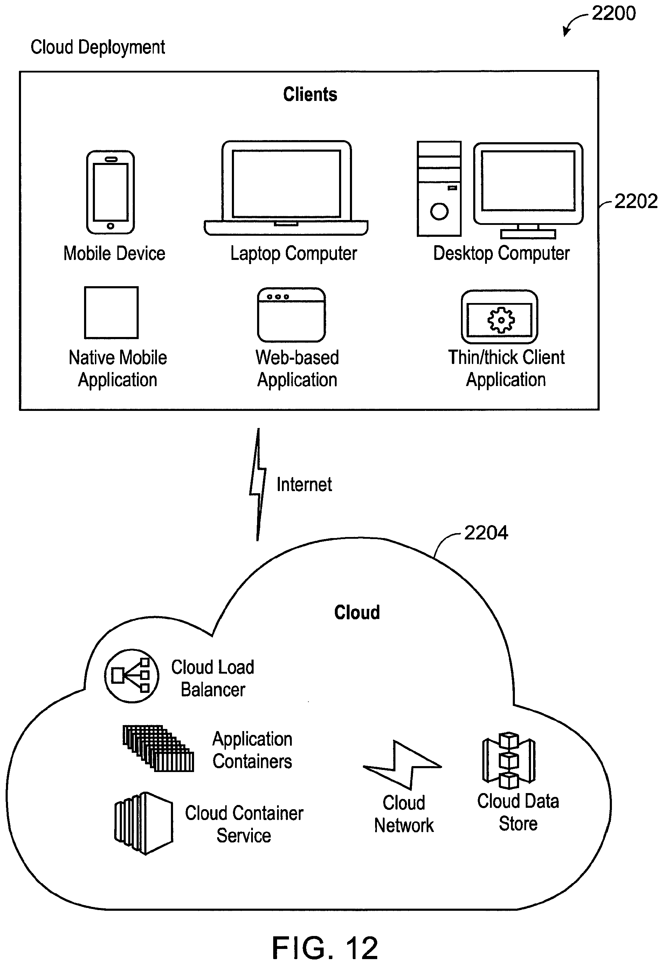

FIG. 12 illustrates a schematic block diagram of another example computing environment 2200 in accordance with this disclosure in which the subject systems, methods and computer readable media can be deployed. The computing environment 2200 includes a cloud deployment architecture consisting of one or more clients 2202 that can be communicatively coupled to a system cloud 2204 via a network (e.g., the Internet). The system cloud 2204 can include a cloud load balances, one or more application container, one or more cloud service containers, a cloud data store, and a cloud network that communicatively couples the one or more cloud components to the cloud data store. In accordance with the cloud deployment architecture, the clients 2202 can include one or more clients devices (e.g., a mobile device, a laptop computer, a desktop computer, etc.) which can include or employ a suitable application (e.g., a native mobile application, a web-based application, a thin/thick client application, etc.) to access and employ one or more features and functionalities of the subject native/reconstructed medical imaging systems deployed in the system cloud 2204. In various implementations, the one or more components of system 100 can be distributed between the clients 2202 and the system cloud 2204.

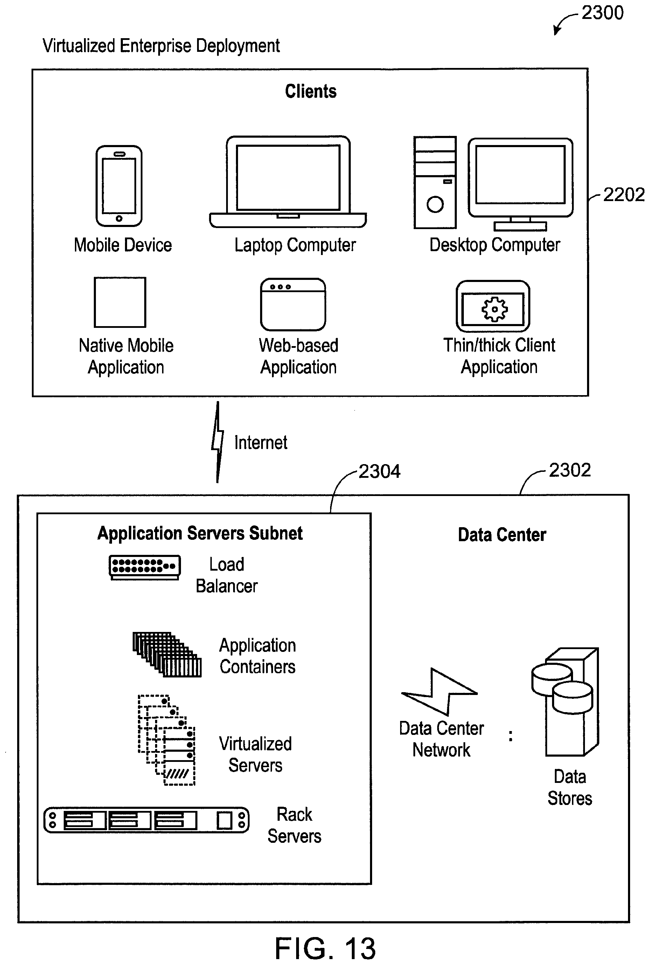

FIG. 13 illustrates a schematic block diagram of another example computing environment 2300 in accordance with this disclosure in which the subject systems, methods and computer readable media can be deployed. The computing environment 2300 includes a virtualized enterprise deployment consisting of one or more clients 2202 that can be communicatively coupled to a remote data center 2302 via a network (e.g., the Internet). The remote data center 2302 can include an application servers subnet 2304 which can provide a load balancer, one or more application containers, one or more virtualized servers and one or more rack servers. The data center 2302 can also include one or more data stores that can be communicatively coupled to the application servers subnet 2304 via a data center network. In accordance with the virtualized enterprise deployment, the clients 2202 can include one or more clients devices (e.g., a mobile device, a laptop computer, a desktop computer, etc.) which can include or employ a suitable application (e.g., a native mobile application, a web-based application, a thin/thick client application, etc.) to access and employ one or more features and functionalities of the subject native/reconstructed medical imaging systems deployed in the data center 2302 and application servers subnet 2304. In various implementations, the one or more components of systems 100 can be distributed between the clients 2202 and the application servers subnet 2304 and the one or more data stores can be provided remotely at the data center 2302.

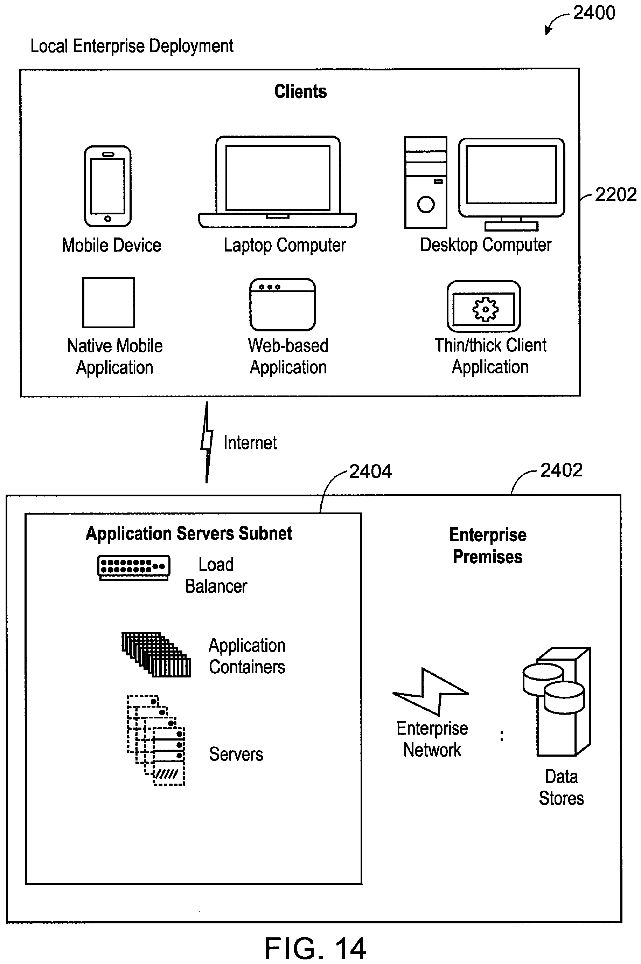

FIG. 14 illustrates a schematic block diagram of another example computing environment 2400 in accordance with this disclosure in which the subject systems, methods and computer readable media can be deployed. The computing environment 2400 includes a local enterprise deployment consisting of one or more clients 2202 that can be communicatively coupled to an application servers subnet 2404 via a network (e.g., the Internet). In accordance with this embodiment, the application servers subnet 2404 can be provided at the enterprise premises 2402 (e.g., as opposed to a remote data center 2302). The application servers subnet 2404 can include a load balancer, one or more application containers and one or more servers. The application servers subnet 2404 can be communicatively coupled to one or more data stores provided at the enterprise premises 2402 via an enterprise network. Similar to the cloud and virtualized enterprise deployments, the clients 2202 can include one or more clients devices (e.g., a mobile device, a laptop computer, a desktop computer, etc.) which can include or employ a suitable application (e.g., a native mobile application, a web-based application, a thin/thick client application, etc.) to access and employ one or more features and functionalities of the subject native/reconstructed medical imaging systems (e.g., system 100and the like) deployed at the enterprise premises 2402 and the application servers subnet 2404. In various implementations, the one or more components of systems herein can be distributed between the clients 2202 and the application servers subnet 2404 and the one or more data stores can be provided at the enterprise premises 2402.

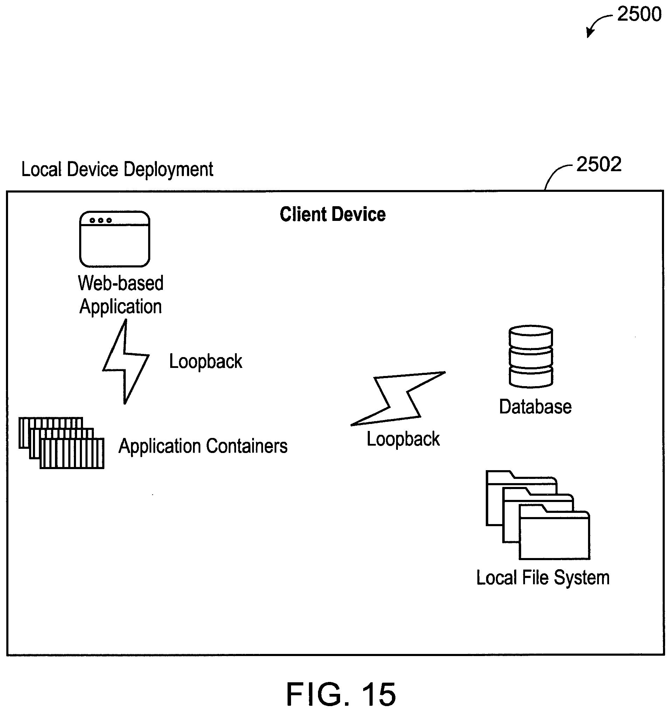

FIG. 15 illustrates a schematic block diagram of another example computing environment in accordance with this disclosure in which the subject systems, methods and computer readable media can be deployed. The computing environment includes a local device deployment in which all of the components of systems herein are provided at a single client device 2502. With this implementation, the client device 2502 can include a web-based application which can be communicatively coupled via a loopback to one or more application containers. The one or more application containers can be communicatively coupled via a loopback to one or more databases and/or one or more local file systems.

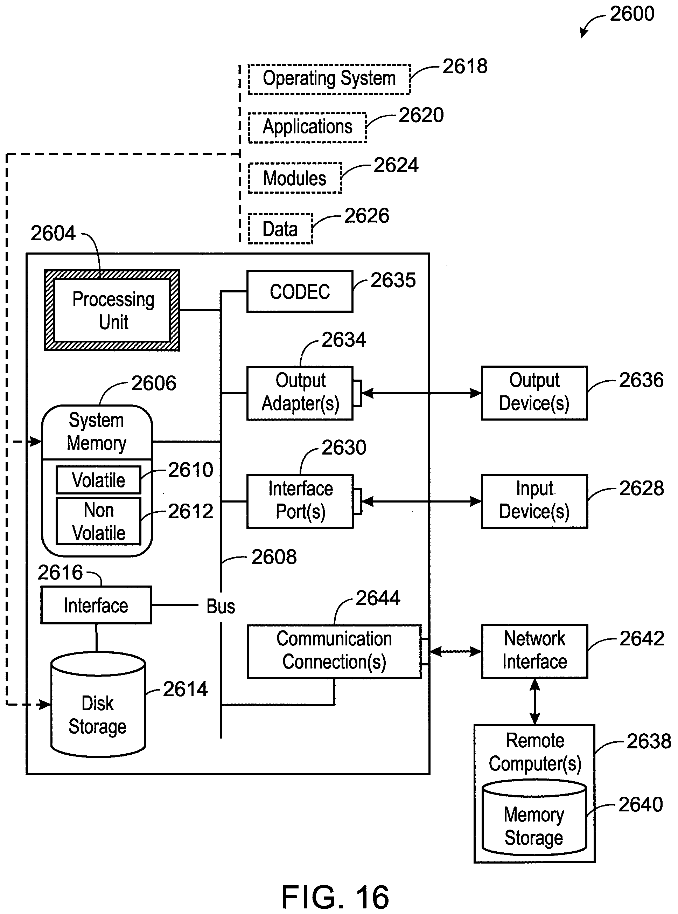

With reference to FIG. 16, a suitable environment 2600 for implementing various aspects of the claimed subject matter includes a computer 2602. The computer 2602 includes a processing unit 2604, a system memory 2606, a codec 2605, and a system bus 2608. The system bus 2608 couples system components including, but not limited to, the system memory 2606 to the processing unit 2604. The processing unit 2604 can be any of various available processors. Dual microprocessors and other multiprocessor architectures also can be employed as the processing unit 2604.

The system bus 2608 can be any of several types of bus structure(s) including the memory bus or memory controller, a peripheral bus or external bus, and/or a local bus using any variety of available bus architectures including, but not limited to, Industrial Standard Architecture (ISA), Micro-Channel Architecture (MSA), Extended ISA (EISA), Intelligent Drive Electronics (IDE), VESA Local Bus (VLB), Peripheral Component Interconnect (PCI), Card Bus, Universal Serial Bus (USB), Advanced Graphics Port (AGP), Personal Computer Memory Card International Association bus (PCMCIA), Firewire (IEEE 22104), and Small Computer Systems Interface (SCSI).

The system memory 2606 includes volatile memory 2610 and non-volatile memory 2612. The basic input/output system (BIOS), containing the basic routines to transfer information between elements within the computer 2602, such as during start-up, is stored in non-volatile memory 2612. In addition, according to present innovations, codec 2605 may include at least one of an encoder or decoder, wherein the at least one of an encoder or decoder may consist of hardware, a combination of hardware and software, or software. Although, codec 2605 is depicted as a separate component, codec 2605 may be contained within non-volatile memory 2612. By way of illustration, and not limitation, non-volatile memory 2612 can include read only memory (ROM), programmable ROM (PROM), electrically programmable ROM (EPROM), electrically erasable programmable ROM (EEPROM), or flash memory. Volatile memory 2210 includes random access memory (RAM), which acts as external cache memory. According to present aspects, the volatile memory may store the write operation retry logic and the like. By way of illustration and not limitation, RAM is available in many forms such as static RAM (SRAM), dynamic RAM (DRAM), synchronous DRAM (SDRAM), double data rate SDRAM (DDR SDRAM), and enhanced SDRAM (ESDRAM).

Computer 2602 may also include removable/non-removable, volatile/non-volatile computer storage medium. FIG. 19 illustrates, for example, disk storage 2614. Disk storage 2614 includes, but is not limited to, devices like a magnetic disk drive, solid state disk (SSD) floppy disk drive, tape drive, Zip drive, flash memory card, or memory stick. In addition, disk storage 2614 can include storage medium separately or in combination with other storage medium including, but not limited to, an optical disk drive such as a compact disk ROM device (CD-ROM), CD recordable drive (CD-R Drive), CD rewritable drive (CD-RW Drive) or a digital versatile disk ROM drive (DVD-ROM). To facilitate connection of the disk storage devices 2614 to the system bus 2608, a removable or non-removable interface is typically used, such as interface 2616.

It is to be appreciated that FIG. 16 describes software that acts as an intermediary between users and the basic computer resources described in the suitable operating environment 2600. Such software includes an operating system 2618. Operating system 2618, which can be stored on disk storage 2614, acts to control and allocate resources of the computer system 2602. Applications 2620 take advantage of the management of resources by operating system 2618 through program modules 2624, and program data 2626, such as the boot/shutdown transaction table and the like, stored either in system memory 2606 or on disk storage 2614. It is to be appreciated that the claimed subject matter can be implemented with various operating systems or combinations of operating systems.

A user enters commands or information into the computer 2602 through input device(s) 2628. Input devices 2628 include, but are not limited to, a pointing device such as a mouse, trackball, stylus, touch pad, keyboard, microphone, joystick, game pad, satellite dish, scanner, TV tuner card, digital camera, digital video camera, web camera, microphone, and the like. These and other input devices connect to the processing unit 2604 through the system bus 2608 via interface port(s) 2630. Interface port(s) 2630 include, for example, a serial port, a parallel port, a game port, and a universal serial bus (USB). Output device(s) 2636 use some of the same type of ports as input device(s). Thus, for example, a USB port may be used to provide input to computer 2602, and to output information from computer 2602 to an output device 2636. Output adapter 2634 is provided to illustrate that there are some output devices 2636 like monitors, speakers, and printers, among other output devices 2636, which require special adapters. The output adapters 2634 include, by way of illustration and not limitation, video and sound cards that provide a means of connection between the output device 2636 and the system bus 2608. It should be noted that other devices and/or systems of devices provide both input and output capabilities such as remote computer(s) 2638.

Computer 2602 can operate in a networked environment using logical connections to one or more remote computers, such as remote computer(s) 2638. The remote computer(s) 2638 can be a personal computer, a server, a router, a network PC, a workstation, a microprocessor based appliance, a peer device, a smart phone, a tablet, or other network node, and typically includes many of the elements described relative to computer 2602. For purposes of brevity, only a memory storage device 2640 is illustrated with remote computer(s) 2638. Remote computer(s) 2638 is logically connected to computer 2602 through a network interface 2642 and then connected via communication connection(s) 2644. Network interface 2642 encompasses wire and/or wireless communication networks such as local-area networks (LAN) and wide-area networks (WAN) and cellular networks. LAN technologies include Fiber Distributed Data Interface (FDDI), Copper Distributed Data Interface (CDDI), Ethernet, Token Ring and the like. WAN technologies include, but are not limited to, point-to-point links, circuit switching networks like Integrated Services Digital Networks (ISDN) and variations thereon, packet switching networks, and Digital Subscriber Lines (DSL).

Communication connection(s) 2644 refers to the hardware/software employed to connect the network interface 2642 to the bus 2608. While communication connection 2644 is shown for illustrative clarity inside computer 2602, it can also be external to computer 2602. The hardware/software necessary for connection to the network interface 2642 includes, for exemplary purposes only, internal and external technologies such as, modems including regular telephone grade modems, cable modems and DSL modems, ISDN adapters, and wired and wireless Ethernet cards, hubs, and routers.

What has been described above includes examples of the embodiments of the present invention. It is, of course, not possible to describe every conceivable combination of components or methodologies for purposes of describing the claimed subject matter, but it is to be appreciated that many further combinations and permutations of the subject innovation are possible. Accordingly, the claimed subject matter is intended to embrace all such alterations, modifications, and variations that fall within the spirit and scope of the appended claims. Moreover, the above description of illustrated embodiments of the subject disclosure, including what is described in the Abstract, is not intended to be exhaustive or to limit the disclosed embodiments to the precise forms disclosed. While specific embodiments and examples are described in this disclosure for illustrative purposes, various modifications are possible that are considered within the scope of such embodiments and examples, as those skilled in the relevant art can recognize.

In particular and in regard to the various functions performed by the above described components, devices, circuits, systems and the like, the terms used to describe such components are intended to correspond, unless otherwise indicated, to any component which performs the specified function of the described component (e.g., a functional equivalent), even though not structurally equivalent to the disclosed structure, which performs the function in the disclosure illustrated exemplary aspects of the claimed subject matter. In this regard, it will also be recognized that the innovation includes a system as well as a computer-readable storage medium having computer-executable instructions for performing the acts and/or events of the various methods of the claimed subject matter.

The aforementioned systems/circuits/modules have been described with respect to interaction between several components/blocks. It can be appreciated that such systems/circuits and components/blocks can include those components or specified sub-components, some of the specified components or sub-components, and/or additional components, and according to various permutations and combinations of the foregoing. Sub-components can also be implemented as components communicatively coupled to other components rather than included within parent components (hierarchical). Additionally, it should be noted that one or more components may be combined into a single component providing aggregate functionality or divided into several separate sub-components, and any one or more middle layers, such as a management layer, may be provided to communicatively couple to such sub-components in order to provide integrated functionality. Any components described in this disclosure may also interact with one or more other components not specifically described in this disclosure but known by those of skill in the art.

In addition, while a particular feature of the subject innovation may have been disclosed with respect to only one of several implementations, such feature may be combined with one or more other features of the other implementations as may be desired and advantageous for any given or particular application. Furthermore, to the extent that the terms "includes," "including," "has," "contains," variants thereof, and other similar words are used in either the detailed description or the claims, these terms are intended to be inclusive in a manner similar to the term "comprising" as an open transition word without precluding any additional or other elements.

As used in this application, the terms "component," "system," or the like are generally intended to refer to a computer-related entity, either hardware (e.g., a circuit), a combination of hardware and software, software, or an entity related to an operational machine with one or more specific functionalities. For example, a component may be, but is not limited to being, a process running on a processor (e.g., digital signal processor), a processor, an object, an executable, a thread of execution, a program, and/or a computer. By way of illustration, both an application running on a controller and the controller can be a component. One or more components may reside within a process and/or thread of execution and a component may be localized on one computer and/or distributed between two or more computers. Further, a "device" can come in the form of specially designed hardware; generalized hardware made specialized by the execution of software thereon that enables the hardware to perform specific function; software stored on a computer readable storage medium; software transmitted on a computer readable transmission medium; or a combination thereof.

Moreover, the words "example" or "exemplary" are used in this disclosure to mean serving as an example, instance, or illustration. Any aspect or design described in this disclosure as "exemplary" is not necessarily to be construed as preferred or advantageous over other aspects or designs. Rather, use of the words "example" or "exemplary" is intended to present concepts in a concrete fashion. As used in this application, the term "or" is intended to mean an inclusive "or" rather than an exclusive "or". That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. In addition, the articles "a" and "an" as used in this application and the appended claims should generally be construed to mean "one or more" unless specified otherwise or clear from context to be directed to a singular form.

Computing devices typically include a variety of media, which can include computer-readable storage media and/or communications media, in which these two terms are used in this description differently from one another as follows. Computer-readable storage media can be any available storage media that can be accessed by the computer, is typically of a non-transitory nature, and can include both volatile and nonvolatile media, removable and non-removable media. By way of example, and not limitation, computer-readable storage media can be implemented in connection with any method or technology for storage of information such as computer-readable instructions, program modules, structured data, or unstructured data. Computer-readable storage media can include, but are not limited to, RAM, ROM, EEPROM, flash memory or other memory technology, CD-ROM, digital versatile disk (DVD) or other optical disk storage, magnetic cassettes, magnetic tape, magnetic disk storage or other magnetic storage devices, or other tangible and/or non-transitory media which can be used to store desired information. Computer-readable storage media can be accessed by one or more local or remote computing devices, e.g., via access requests, queries or other data retrieval protocols, for a variety of operations with respect to the information stored by the medium.

In view of the exemplary systems described above, methodologies that may be implemented in accordance with the described subject matter will be better appreciated with reference to the flowcharts of the various figures. For simplicity of explanation, the methodologies are depicted and described as a series of acts. However, acts in accordance with this disclosure can occur in various orders and/or concurrently, and with other acts not presented and described in this disclosure. Furthermore, not all illustrated acts may be required to implement the methodologies in accordance with certain aspects of this disclosure. In addition, those skilled in the art will understand and appreciate that the methodologies could alternatively be represented as a series of interrelated states via a state diagram or events. Additionally, it should be appreciated that the methodologies disclosed in this disclosure are capable of being stored on an article of manufacture to facilitate transporting and transferring such methodologies to computing devices. The term article of manufacture, as used in this disclosure, is intended to encompass a computer program accessible from any computer-readable device or storage media

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description. The scope of the various embodiments of the invention should, therefore, be determined with reference to the appended claims, along with the full scope of equivalents to which such claims are entitled.

In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects. Further, the limitations of the following claims are not written in means-plus-function format and are not intended to be interpreted based on 35 U.S.C. § 112, sixth paragraph, unless and until such claim limitations expressly use the phrase "means for" followed by a statement of function void of further structure.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

## Claims

1. A healthcare system, comprising:
one or more processors (2604);
memory (2606) ; and
one or more programs, wherein the one or more programs are stored in the memory (2606) and configured to be executed by the one or more processors (2604), the one or more programs including instructions for:
patient intake (412, 500) utilizing a user interface (116), wherein patient intake (412, 500) additionally receives information from disparate information sources comprising at least an electronic medical record (EMR) and a picture archival and communications system (PACS) and can pre-populate parts of the patient intake form based on artificial intelligence analysis of the information from the disparate information sources;
workflow management through a medical diagnosis and treatment process based on the patient intake (412, 500), including providing a user interface (116) displaying the workflow orchestration across the diagnosis and treatment process;
trend monitoring (604) for each step in the workflow management, wherein the trend monitoring (604) may provide suggestions in the workflow based on automatic quality reviews related to the trend; and
treatment planning (484) based on user input during the workflow of the medical diagnosis and treatment process.

2. The healthcare system of claim 1, wherein:
the automatic quality reviews related to the trend are based on a medical industry quality standard.

3. The healthcare system of claim 1, wherein the one or more programs further include instructions for:
an interoperability engine (112) providing operability between various software applications and data storage systems based on industry standards and application specific programming interfaces.

4. The healthcare system of claim 1, wherein:
treatment planning (484) may be further based on predictive analysis on the data in the system based on the patient intake (412, 500) and workflow management, stored in a non-relational database and utilizing a Bayesian statistics method of predictive analysis.

5. The healthcare system of claim 1, wherein:
the artificial intelligence analysis of the information from the disparate information sources includes checking the information based on the possible bounds for the related field of the patient intake form.

6. The healthcare system of claim 1, wherein:
the artificial intelligence analysis of the information from the disparate information sources includes searching and matching the patient (24) info with related clinical trials and verifying the patient (24) info fits within the clinical trial guidelines.

7. The healthcare system of claim 1, wherein the one or more programs further include instructions for:
pattern recognition (910) between patients based on artificial intelligence analysis of the workflow management of a plurality of patients and providing user interface (116) suggestions on a display based on recognized patterns.

8. A healthcare method, comprising: at a device having one or more processors (2604) and memory (2606), including the steps of:
patient intake (412, 500) utilizing a user interface (116), wherein patient intake (412, 500) additionally receives information from disparate information sources comprising at least an electronic medical record (EMR) and a picture archival and communications system (PACS) and can pre-populate parts of the patient intake form based on artificial intelligence analysis of the information from the disparate information sources;
workflow management through a medical diagnosis and treatment process based on the patient intake (412, 500), including providing a user interface (116) displaying the workflow orchestration across the diagnosis and treatment process;
trend monitoring (604) for each step in the workflow management, wherein the trend monitoring (604) may provide suggestions in the workflow based on automatic quality reviews related to the trend; and
treatment planning (484) based on user input during the workflow of the medical diagnosis and treatment process.

9. The healthcare method of claim 8, wherein:
the automatic quality reviews related to the trend are based on a medical industry quality standard.

10. The healthcare method of claim 8, wherein the one or more programs further include steps of:
an interoperability engine (112) providing operability between various software applications and data storage systems based on industry standards and application specific programming interfaces.

11. The healthcare method of claim 8, wherein:
treatment planning (484) may be further based on predictive analysis on the data in the system based on the patient intake (412, 500) and workflow management, stored in a non-relational database and utilizing a Bayesian statistics method of predictive analysis.

12. The healthcare method of claim 8, wherein:
the artificial intelligence analysis of the information from the disparate information sources includes checking the information based on the possible bounds for the related field of the patient intake form.

13. The healthcare method of claim 8, wherein:
the artificial intelligence analysis of the information from the disparate information sources includes searching and matching the patient info with related clinical trials and verifying the patient info fits within the clinical trial guidelines.

14. The healthcare method of claim 8, wherein the one or more programs further include steps of:
pattern recognition (910) between patients based on artificial intelligence analysis of the workflow management of a plurality of patients and providing user interface (116) suggestions on a display based on recognized patterns.

15. A healthcare engine embodied on a non-transitory computer readable storage medium storing one or more programs, the one or more programs comprising instructions, which when executed by one or more processors (2604) of an electronic device, cause the device to provide:
patient intake (412, 500) utilizing a user interface (116), wherein patient intake (412, 500) additionally receives information from disparate information sources comprising at least an electronic medical record (EMR) and a picture archival and communications system (PACS) and can pre-populate parts of the patient intake form based on artificial intelligence analysis of the information from the disparate information sources;
workflow management through a medical diagnosis and treatment process based on the patient intake (412, 500), including providing a user interface (116) displaying the workflow orchestration across the diagnosis and treatment process;
trend monitoring (604) for each step in the workflow management, wherein the trend monitoring (604) may provide suggestions in the workflow based on automatic quality reviews related to the trend; and
treatment planning (484) based on user input during the workflow of the medical diagnosis and treatment process.

16. The healthcare engine of claim 15, wherein the one or more programs further include instructions for:
an interoperability engine (112) providing operability between various software applications and data storage systems based on industry standards and application specific programming interfaces.

17. The healthcare engine of claim 15, wherein:
treatment planning (484) may be further based on predictive analysis on the data in the system based on the patient intake (412, 500) and workflow management, stored in a non-relational database and utilizing a Bayesian statistics method of predictive analysis.

18. The healthcare engine of claim 15, wherein:
the artificial intelligence analysis of the information from the disparate information sources includes checking the information based on the possible bounds for the related field of the patient intake form.

19. The healthcare engine of claim 15, wherein:
the artificial intelligence analysis of the information from the disparate information sources includes searching and matching the patient (24) info with related clinical trials and verifying the patient (24) info fits within the clinical trial guidelines.

20. The healthcare engine of claim 15, wherein the one or more programs further include instructions for:
pattern recognition (910) between patients based on artificial intelligence analysis of the workflow management of a plurality of patients and providing user interface (116) suggestions on a display based on recognized patterns.
